(19) Europäisches Patentamt
European Patent Office
Office européen des brevets

(11) EP 4 506 347 A1

(12) EUROPEAN PATENT APPLICATION
published in accordance with Art. 153(4) EPC

(43) Date of publication:
**12.02.2025 Bulletin 2025/07**

(21) Application number: **23780672.4**

(22) Date of filing: **29.03.2023**

(51) International Patent Classification (IPC):
***C07D 471/04*** *(2006.01)* ***A61K 31/5513*** *(2006.01)*
***A61P 27/02*** *(2006.01)* ***A61P 27/06*** *(2006.01)*

(52) Cooperative Patent Classification (CPC):
**A61K 31/5513; A61P 27/02; A61P 27/06; C07D 471/04**

(86) International application number:
**PCT/JP2023/012761**

(87) International publication number:
**WO 2023/190663 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.03.2022 JP 2022056754**

(71) Applicant: **Santen Pharmaceutical Co., Ltd.**
**Kita-ku**
**Osaka-shi**
**Osaka 530-8552 (JP)**

(72) Inventors:
- **OHNO, Atsushi**
  **Ikoma-shi, Nara 630-0101 (JP)**
- **HONDA, Takahiro**
  **Ikoma-shi, Nara 630-0101 (JP)**

(74) Representative: **Müller-Boré & Partner Patentanwälte PartG mbB**
**Friedenheimer Brücke 21**
**80639 München (DE)**

# (54) HIGH-PURITY COMPOUND PRODUCTION METHOD AND PURIFICATION METHOD

(57) The present invention provides an effective preparation method and/or an effective purification method of (R)-11-[2-[2-[(diethylamino)methyl]-1-piperidinyl]acetyl]-5,11-dihydro-6H-pyrido[2,3-b][1,4]benzodiazepin-6-one or a salt thereof in high yield and high purity which is free of impurities, particularly 11-(2-chloroacetyl)-5,11-dihydro-6H-pyrido[2,3-b][1,4]benzodiazepin-6-one.



Processed by Luminess, 75001 PARIS (FR)

## Description

### TECHNICAL FIELD

The present invention relates to a preparation method and a purification method of (R)-11-[2-[2-[(diethylamino)methyl]-1-piperidinyl]acetyl]-5,11-dihydro-6H-pyrido[2,3-b][1,4]benzodiazepin-6-one (hereinafter also referred to as "AFDX0250") or a salt thereof in high purity. Also, the present invention relates to AFDX0250 or a salt thereof in high purity and a pharmaceutical composition comprising AFDX0250 or a salt thereof in high purity wherein the pharmaceutical composition is substantially free of impurities such as 11-(2-chloroacetyl)-5,11-dihydro-6H-pyrido[2,3-b][1,4]benzodiazepin-6-one (hereinafter also referred to as "Compound 1").

### BACKGROUND ART

It has been known that AFDX0250 is (+)-11-[2-[2-[(diethylamino)methyl]-1-piperidinyl]acetyl]-5,11-dihydro-6H-pyrido[2,3-b][1,4]benzodiazepin-6-one having the following structure and is useful for the treatment of an eye disease such as glaucoma, ocular hypertension and myopia (Patent Document 1 and Patent Document 2).

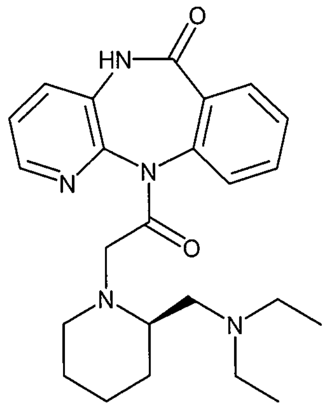

The preparation method of AFDX0250 is disclosed in Non-Patent Document 1 and Patent Document 3. Non-Patent Document 1 discloses that according to the general procedure of reacting in acetonitrile in the presence of sodium carbonate, 11-(chloroacetyl)-5,11-dihydro-6H-pyrido[2,3-b][1 ,4]benzodiazepin-6-one and (-)-2-[(diethylamino)methyl]piperidine are reacted to prepare AFDX0250. Patent Document 3 also discloses that according to the example of reacting in acetonitrile in the presence of sodium carbonate, 11-(chloroacetyl)-5,11-dihydro-6H-pyrido[2,3-b][1,4]benzodiazepin-6-one and (-)-2-[(diethylamino)methyl]piperidine are reacted to prepare AFDX0250, but the yield of AFDX0250 is as low as 59%.

Hence, it is important to manufacture AFDX0250 in high yield and effectively purify the manufactured AFDX0250 in order to manufacture and provide AFDX0250 as a pharmaceutical product as well as to remove impurities from the crude product and manufacture AFDX0250 in high purity to avoid undesirable effects.

### PRIOR ART DOCUMENTS

### PATENT DOCUMENTS

Patent Document 1: WO 93/18772
Patent Document 2: WO 2022/030489
Patent Document 3: JP S60-215683

### NON-PATENT DOCUMENTS

Non-Patent Document 1: Wolfnard W. Engel et al., J. Med. Chem., 1989, 32(8), 1718-1724

## SUMMARY OF THE INVENTION

### PROBLEMS TO BE SOLVED BY THE INVENTION

Pharmaceutical products are required to have high purity to comply with the standards of approval for quality and purity. For example, when impurities are produced during the manufacture and/or storage of pharmaceutical products, the produced impurities may cause problems that the therapeutic effects of the pharmaceutical products are reduced and undesirable effects such as side effects are produced. In addition, when the yield of a compound useful as an active ingredient in pharmaceutical products is low, it is difficult to manufacture the compound in large amounts or to produce the compound industrially. As a result, it causes the problem of high cost in the development of pharmaceutical products. As described above, Non-Patent Document 1 and Patent Document 3 disclose a preparation method of AFDX0250. On the other hand, it is disclosed that the yield of the prepared AFDX0250 is low. Hence, there is room for improvement of the conventional preparation methods of AFDX0250 to use AFDX0250 as pharmaceutical products.

In addition, the present inventors have found that 11-(2-chloroacetyl)-5,11-dihydro-6H-pyrido[2,3-b] [1,4]benzodiazepin-6-one contained in the prepared AFDX0250 is positive in Ames test in the development stage of a pharmaceutical composition comprising AFDX0250 or a salt thereof. As a result, they have thought that it is desirable to remove the compound from the viewpoint of the expression of side effects.

An object of the present invention is to provide an effective preparation method and an effective purification method of AFDX0250 in high yield and high purity which is free of impurities, particularly 11-(2-chloroacetyl)-5,11-dihydro-6H-pyrido[2,3-b][1,4]benzodiazepin-6-one. In addition, another object of the present invention is to provide AFDX0250 or a salt thereof in high yield and high purity which is substantially free of impurities and a pharmaceutical composition comprising AFDX0250 in high purity with high safety.

### SOLUTIONS TO THE PROBLEMS

The present inventors have studied to solve the objects and have found a specific condition for preparing AFDX0250 or a salt thereof in high yield and high purity from 11-(2-chloroacetyl)-5,11-dihydro-6H-pyrido[2,3-b][1,4]benzodiazepin-6-one or a salt thereof and (R)-2-(diethylamino)methylpiperidine or a salt thereof as well as a specific condition of the crystallization for purifying AFDX0250 or a salt thereof in high purity. More specifically, the present inventors have found that AFDX0250 or a salt thereof in high yield and high purity can be prepared by reacting 11-(2-chloroacetyl)-5,11-dihydro-6H-pyrido[2,3-b][1,4]benzodiazepin-6-one or a salt thereof and (R)-2-(diethylamino)methylpiperidine or a salt thereof in an organic solvent comprising acetonitrile in an amount of 15 to 50 mL per g of 11-(2-chloroacetyl)-5,11-dihydro-6H-pyrido[2,3-b][1,4]benzodiazepin-6-one or a salt thereof under a basic condition in the presence of an alkali metal iodide and that the resulting crude product of AFDX0250 can be crystallized to higher purity by appropriately adjusting the solvent and temperature used. Based on the findings, the present invention has been completed.

Specifically, the present invention provides the following embodiments.

[Item 1] A method of preparing (R)-11-[2-[2-[(diethylamino)methyl]-1-piperidinyl]acetyl]-5,11-dihydro-6H-pyrido[2,3-b][1,4]benzodiazepin-6-one or a salt thereof, which comprises reacting 11-(2-chloroacetyl)-5,11-dihydro-6H-pyrido[2,3-b][1,4]benzodiazepin-6-one or a salt thereof and (R)-2-(diethylamino)methylpiperidine or a salt thereof in an organic solvent under a basic condition in the presence of an alkali metal iodide, wherein the organic solvent comprises acetonitrile in an amount of 15 to 50 mL per g of 11-(2-chloroacetyl)-5,11-dihydro-6H-pyrido[2,3-b][1,4]benzodiazepin-6-one or a salt thereof.

[Item 2] The method according to the item 1, wherein the organic solvent is acetonitrile in an amount of 15 to 50 mL per g of 11-(2-chloroacetyl)-5,11-dihydro-6H-pyrido[2,3-b][1,4]benzodiazepin-6-one or a salt thereof.

[Item 3] The method according to the item 1 or 2, which comprises adding water and an acid to the resulting reaction mixture to adjust the pH thereof and then extracting the reaction mixture with an organic solvent.

[Item 4] The method according to the item 3, wherein the extraction with an organic solvent is performed twice or more.

[Item 5] The method according to the item 3 or 4, wherein the organic solvent is ethyl acetate.

[Item 6] A method of preparing (R)-11-[2-[2-[(diethylamino)methyl]-1-piperidinyl]acetyl]-5,11-dihydro-6H-pyrido[2,3-b][1,4]benzodiazepin-6-one or a salt thereof, which comprises reacting 11-(2-iodoacetyl)-5,11-dihydro-6H-pyrido[2,3-b][1,4]benzodiazepin-6-one or a salt thereof and (R)-2-(diethylamino)methylpiperidine or a salt thereof in an organic solvent under a basic condition.

[Item 7] A method of purifying (R)-11-[2-[2-[(diethylamino)methyl]-1-piperidinyl]acetyl]-5,11-dihydro-6H-pyrido[2,3-b][1,4]benzodiazepin-6-one or a salt thereof, which comprises crystallizing (R)-11-[2-[2-[(diethylamino)methyl]-1-piperidinyl]acetyl]-5,11-dihydro-6H-pyrido[2,3-b][1,4]benzodiazepin-6-one or a salt thereof in a mixture of 1-propanol and water.

[Item 8] A method of purifying (R)-11-[2-[2-[(diethylamino)methyl]-1-piperidinyl]acetyl]-5,11-dihydro-6H-pyrido[2,3-b][1,4]benzodiazepin-6-one or a salt thereof, which comprises treating (R)-11-[2-[2-[(diethylamino)methyl]-1-piperidinyl]acetyl]-5,11-dihydro-6H-pyrido[2,3-b][1,4]benzodiazepin-6-one or a salt thereof with an activated carbon in an alcohol solvent.

[Item 9] A method of purifying (R)-11-[2-[2-[(diethylamino)methyl]-1-piperidinyl]acetyl]-5,11-dihydro-6H-pyrido[2,3-b][1,4]benzodiazepin-6-one or a salt thereof, which comprises recrystallizing (R)-11-[2-[2-[(diethylamino)methyl]-1-piperidinyl]acetyl]-5,11-dihydro-6H-pyrido[2,3-b][1,4]benzodiazepin-6-one or a salt thereof in a mixture of an alcohol solvent and a ketone solvent.

[Item 10] (R)-11-[2-[2-[(diethylamino)methyl]-1-piperidinyl]acetyl]-5,11-dihydro-6H-pyrido[2,3-b][1,4]benzodiazepin-6-one or a salt thereof with a purity of 95% or more.

[Item 11] (R)-11-[2-[2-[(diethylamino)methyl]-1-piperidinyl]acetyl]-5,11-dihydro-6H-pyrido[2,3-b][1,4]benzodiazepin-6-one or a salt thereof, wherein an amount of impurities therein is less than 5%.

[Item 12] A pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt, wherein the pharmaceutical composition comprises impurities derived from (R)-11-[2-[2-[(diethylamino)methyl]-1-piperidinyl]acetyl]-5,11-dihydro-6H-pyrido[2,3-b] [1,4] benzodiazepin-6-one or a salt thereof in an amount of less than 5% relative to (R)-11-[2-[2-[(diethylamino)methyl]-1-piperidinyl]acetyl]-5,11-dihydro-6H-pyrido[2,3-b][1,4]benzodiazepin-6-one or a salt thereof.

[Item 13] The pharmaceutical composition according to the item 12, wherein the amount of the impurities is less than 1%.

[Item 14] A pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the pharmaceutical composition is substantially free of 11-(2-chloroacetyl)-5,11-dihydro-6H-pyrido[2,3-b][1,4]benzodiazepin-6-one or a salt thereof.

[Item 15] The pharmaceutical composition according to the item 14, wherein the amount of 11-(2-chloroacetyl)-5,11-dihydro-6H-pyrido[2,3-b][1,4]benzodiazepin-6-one or a salt thereof is less than 1000 ppm relative to (R)-11-[2-[2-[(diethylamino)methyl]-1-piperidinyl]acetyl]-5,11-dihydro-6H-pyrido[2,3-b][1,4]benzodiazepin-6-one or a salt thereof.

[Item 16] The pharmaceutical composition according to the item 14 or 15, wherein the pharmaceutical composition is substantially free of 5,11-dihydro-6H-pyrido[2,3-b][1,4]benzodiazepin-6-one or a salt thereof.

[Item 17] The pharmaceutical composition according to the item 16, wherein the amount of 5,11-dihydro-6H-pyrido[2,3-b][1,4]benzodiazepin-6-one or a salt thereof is less than 1000 ppm.

Each feature of the above [Item 1] to [Item 17] may be optionally selected and combined two or more.

The present invention further provides the following embodiments.

[Item 18] The pharmaceutical composition according to any one of the items 12 to 17 which is an eye drop.

[Item 19] The pharmaceutical composition according to any one of the items 12 to 18 for treating glaucoma, ocular hypertension, or myopia.

## EFFECTS OF THE INVENTION

According to the preparation method and the purification method of the present invention, (R)-11-[2-[2-[(diethylamino)methyl]-1-piperidinyl]acetyl]-5,11-dihydro-6H-pyrido[2,3-b][1,4]benzodiazepin-6-one or a salt thereof in higher yield and higher purity can be prepared as compared to known methods. Also, the present invention can reduce the amount of impurities such as 11-(2-chloroacetyl)-5,11-dihydro-6H-pyrido[2,3-b][1,4]benzodiazepin-6-one.

In addition, the present invention can provide (R)-11-[2-[2-[(diethylamino)methyl]-1-piperidinyl]acetyl]-5,11-dihydro-6H-pyrido[2,3-b][1,4]benzodiazepin-6-one or a salt thereof in high purity and a pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof in high purity.

## BRIEF DESCRIPTION OF THE DRAWINGS

[Fig. 1] Fig. 1 shows the appearances of AFDX0250 samples (powders and DMSO solutions) comprising each solvent (ethanol (EtOH), 1-propanol (1-PrOH) and N-methylpyrrolidone (NMP)) before and after the treatment with activated carbon, Shirasagi A (0.05 w/w, 0.10 w/w and 0.15 w/w) and Shirasagi P (0.10 w/w and 0.15 w/w). Samples comprising NMP as solvent treated with Shirasagi A (0.15 w/w) and Shirasagi P (0.10 w/w and 0.15 w/w) are not prepared.

[Fig. 2] Fig. 2 shows the appearances of each dried product and DMSO solution prepared by the recrystallization with

each combination of ethanol as an alcohol solvent and acetone, methyl ethyl ketone (MEK), methyl isobutyl ketone (MIBK) or n-heptane as a poor solvent at total solvent amounts of 10 v/w (Ratio 1:3) and 20 v/w (Ratio 1:1) as well as dried product and DMSO solution before purification.

[Fig. 3] Fig. 3 shows the appearances of each dried product and DMSO solution prepared by the recrystallization with each combination of 1-propanol as an alcohol solvent and acetone, methyl ethyl ketone (MEK), methyl isobutyl ketone (MIBK) or n-heptane as a poor solvent at total solvent amounts of 10 v/w (Ratio 1:3) and 20 v/w (Ratio 1:1) as well as dried product and DMSO solution before purification.

## DETAILED DESCRIPTION

Hereinafter, the present invention is illustrated in detail.

The present invention provides a method of preparing (R)-11-[2-[2-[(diethylamino)methyl]-1-piperidinyl]acetyl]-5,11-dihydro-6H-pyrido[2,3-b][1,4]benzodiazepin-6-one or a salt thereof. The method comprises reacting 11-(2-chloroacetyl)-5,11-dihydro-6H-pyrido[2,3-b][1,4]benzodiazepin-6-one or a salt thereof and (R)-2-(diethylamino)methylpiperidine or a salt thereof in an organic solvent including acetonitrile, preferably acetonitrile under a basic condition in the presence of an alkali metal iodide. In the process, the reaction mixture may be prepared by mixing 11-(2-chloroacety1)-5,11-dihydro-6H-pyrido[2,3-b][1,4]benzodiazepin-6-one or a salt thereof, (R)-2-(diethylamino)methylpiperidine or a salt thereof, an organic solvent including acetonitrile, preferably acetonitrile, an alkali metal iodide and a base, heating and stirring the mixture, and then concentrating the mixture.

The starting materials in the process, 11-(2-chloroacetyl)-5,11-dihydro-6H-pyrido[2,3-b][1,4]benzodiazepin-6-one or a salt thereof and (R)-2-(diethylamino)methylpiperidine or a salt thereof may be commercially available products or may be prepared by any well-known methods.

The "organic solvent" in the process is an organic solvent including acetonitrile, preferably acetonitrile alone. The amount of acetonitrile added is 15 v/w to 50 v/w, preferably 15 v/w to 40 v/w, more preferably 15 v/w to 30 v/w, furthermore preferably 18 v/w to 25 v/w, and most preferably 20 v/w.

The term "v/w" in the amount of acetonitrile added means the volume (mL) of acetonitrile relative to the mass (1 g) of 11-(2-chloroacetyl)-5,11-dihydro-6H-pyrido[2,3-b][1,4]benzodiazepin-6-one. Hereinafter, the same shall apply to the followings unless otherwise specified.

Examples of the "alkali metal iodide" in the process include sodium iodide, potassium iodide, calcium iodide, lithium iodide, and others. It is preferably sodium iodide. Also, the alkali metal iodide may be used alone or two or more alkali metal iodides may be used in any combination and ratio. The amount of the alkali metal iodide added is, for example, 0.01 to 2 equivalents, preferably 0.05 to 1.5 equivalents, more preferably 0.1 to 1 equivalent, furthermore preferably 0.15 to 0.5 equivalent, particularly preferably 0.2 to 0.4 equivalent, and most preferably 0.25 equivalent.

The term "equivalent" in the amount of the alkali metal iodide added means the amount (mol) of an alkali metal iodide relative to the amount (1 mol) of 11-(2-chloroacetyl)-5,11-dihydro-6H-pyrido[2,3-b][1,4]benzodiazepin-6-one. Hereinafter, the same shall apply to the followings unless otherwise specified.

Examples of the "basic condition" in the process include basic conditions with lithium carbonate, sodium carbonate, cesium carbonate, potassium carbonate, lithium hydrogen carbonate, sodium hydrogen carbonate, potassium hydrogen carbonate, lithium hydride, sodium hydride, potassium hydride, lithium hydroxide, sodium hydroxide, potassium hydroxide, lithium *tert*-butoxide, sodium *tert*-butoxide, potassium *tert*-butoxide, alkyllithium (e.g., normal-butyllithium, *sec*-butyllithium, *tert*-butyllithium, *normal*-hexyllithium), lithium amide (e.g., lithium diisopropylamide, lithium hexamethyldisilazide), sodium methoxide, sodium ethoxide, *tert*-amine (e.g., trimethylamine, triethylamine, tripropylamine, triisopropylamine, diisopropylethylamine), or others. It is preferably the basic condition with sodium hydrogen carbonate, triethylamine or diisopropylethylamine. The base may be used alone or two or more of the bases may be used in any combination and ratio. The amount of the base added is, for example, 1 to 20 equivalents, preferably 1.5 to 15 equivalents, more preferably 2 to 10 equivalents, furthermore preferably 2.5 to 8 equivalents, particularly preferably 3 to 5 equivalents, and most preferably 3.15 equivalents.

The term "equivalent" in the amount of the base added means the amount (mol) of a base relative to the amount (1 mol) of 11-(2-chloroacetyl)-5,11-dihydro-6H-pyrido[2,3-b][1,4]benzodiazepin-6-one. Hereinafter, the same shall apply to the followings unless otherwise specified.

The reaction temperature in the process is, for example, 40 to 100 °C, preferably 50 to 90 °C, more preferably 60 to 80 °C, furthermore preferably 65 to 78 °C, and most preferably 70 to 75 °C.

The reaction time in the process is not particularly limited because it varies depending on various conditions such as reaction temperature. The reaction time is appropriately selected from, for example, 1 to 48 hours. The reaction time is preferably 3 to 36 hours, more preferably 6 to 30 hours, furthermore preferably 12 to 27 hours, particularly preferably 18 to 24 hours, and most preferably 20 hours.

The preparation method of the present invention comprises concentrating the reaction mixture prepared in the above process to, for example, 3 to 12 v/w, preferably 4 to 10 v/w, more preferably 5 to 8 v/w, most preferably 6 to 7 v/w,

adding water and an acid to adjust the pH of the mixture to a pH at which (R)-11-[2-[2-[(diethylamino)methyl]-1-piperidinyl] acetyl]-5,11-dihydro-6H-pyrido[2,3-b][1,4]benzodiazepin-6-one or a salt thereof can be dissolved in water, and then extracting the mixture with an organic solvent. In the process, to the reaction mixture were added water and then an acid to adjust the pH thereof to acidity, for example, 3.0 or less, more preferably 2.0 or less, and most preferably 1.0 or less, and the mixture is extracted with an organic solvent one or more times, preferably two or more times, more preferably three or more times, and most preferably four or more times.

The term "v/w" in the amount of the reaction mixture means the volume (mL) of the reaction mixture relative to the mass (1 g) of 11-(2-chloroacetyl)-5,11-dihydro-6H-pyrido[2,3-b][1,4]benzodiazepin-6-one.

The amount of water added in the process is, for example, 3 to 50 v/w, preferably 5 to 30 v/w, more preferably 7 to 20 v/w, furthermore preferably 8 to 15 v/w, and most preferably 10 v/w.

The term "v/w" in the amount of water added means the volume (mL) of the water relative to the mass (1 g) of 11-(2-chloroacetyl)-5,11-dihydro-6H-pyrido[2,3-b][1,4]benzodiazepin-6-one. Hereinafter, the same shall apply to the followings unless otherwise specified.

The acid in the process is not particularly limited as long as it is an acid which can adjust the pH to a pH at which (R)-11-[2-[2-[(diethylamino)methyl]-1-piperidinyl]acetyl]-5,11-dihydro-6H-pyrido[2,3-b][1,4]benzodiazepin-6-one or a salt thereof can be dissolved in water. Examples of the acid include hydrochloric acid, sulfuric acid, nitric acid, and others. It is preferably hydrochloric acid. The amount of the acid added is not particularly limited as long as it is an amount which can adjust the pH to a pH at which (R)-11-[2-[2-[(diethylamino)methyl]-1-piperidinyl]acetyl]-5,11-dihydro-6H-pyrido [2,3-b][1,4]benzodiazepin-6-one or a salt thereof can be dissolved in water.

Examples of the organic solvent in the process include chloroform, dichloromethane, diethyl ether, tetrahydrofuran (THF), ethyl acetate, hexane, toluene, and others. It is preferably ethyl acetate. The amount of the organic solvent added is, for example, 5 v/w to 50 v/w, preferably 10 v/w to 40 v/w, and most preferably 10 v/w.

The term "v/w" in the amount of the organic solvent added means the volume (mL) of the organic solvent relative to the mass (1 g) of 11-(2-chloroacetyl)-5,11-dihydro-6H-pyrido[2,3-b][1,4]benzodiazepin-6-one.

The temperature when water and an acid are added in the process is, for example, -10 to 30 °C, preferably -5 to 20 °C, more preferably 0 to 15 °C, and most preferably 0 to 10 °C.

The preparation method of the present invention comprises adding a solvent into the organic layer obtained in the above process and crystallizing the compound therein. In the process, (R)-11-[2-[2-[(diethylamino)methyl]-1-piperidinyl] acetyl]-5,11-dihydro-6H-pyrido[2,3-b][1,4]benzodiazepin-6-one or a salt thereof can be crystallized by adding a solvent to the organic layer, adding a base to adjust the pH of the mixture to alkalinity, preferably 8 to 12, more preferably 9 to 11, and then stirring the mixture. (R)-11-[2-[2-[(Diethylamino)methyl]-1-piperidinyl]acetyl]-5,11-dihydro-6H-pyrido[2,3-b][1,4] benzodiazepin-6-one or a salt thereof in high purity can be obtained by removing the precipitated crystal by filtration, washing, and drying the filtrate.

Examples of the solvent in the process include water, a mixture of methanol and water, a mixture of ethanol and water, a mixture of acetone and water, a mixture of methyl ethyl ketone and water, a mixture of acetonitrile and water, a mixture of N,N-dimethylformamide and water, and others. It is preferably a mixture of acetonitrile and water. When a mixture of acetonitrile and water is used as the solvent, the volume ratio of acetonitrile and water is, for example, 1:1 to 1:10, preferably 1:2 to 1:6, and most preferably 1:3 to 1:5. The amount of the solvent added is, for example, 5 to 20 v/w, preferably 8 to 16 v/w, and more preferably 10 to 14 v/w.

The term "v/w" in the amount of the solvent added means the volume (mL) of the solvent relative to the mass (1 g) of 11-(2-chloroacetyl)-5,11-dihydro-6H-pyrido[2,3-b][1,4]benzodiazepin-6-one.

The base in the process is not particularly limited as long as it is a base which can adjust the pH to alkalinity, preferably 8 to 12, more preferably 9 to 11. Examples of the base include lithium carbonate, sodium carbonate, potassium carbonate, sodium hydroxide, potassium hydroxide, lithium hydroxide, and others. It is preferably potassium hydroxide. The amount of the base added is not particularly limited as long as it is an amount which can adjust the pH to alkalinity, preferably 8 to 12, more preferably 9 to 11.

The temperature when stirring in the process is, for example, -10 to 20 °C, preferably -5 to 15 °C, and most preferably 0 to 10°C.

The stirring time in the process is not particularly limited because it varies depending on various reaction conditions such as temperature. The stirring time is appropriately selected from, for example, 1 to 24 hours. The stirring time is preferably 1 to 12 hours, more preferably 2 to 8 hours, and most preferably 3 to 5 hours.

The drying temperature in the process is not particularly limited as long as it is a temperature range which can remove adhered moisture or solution. The drying temperature is, for example, 30 to 100 °C, preferably about 50 °C.

The preparation method of the present invention can remove 11-(2-chloroacetyl)-5,11-dihydro-6H-pyrido[2,3-b] [1,4]benzodiazepin-6-one or a salt thereof.

The present invention also provides a method of preparing (R)-11-[2-[2-[(diethylamino)methyl]-1-piperidinyl] acetyl]-5,11-dihydro-6H-pyrido[2,3-b][1,4]benzodiazepin-6-one or a salt thereof, which comprises reacting 11-(2-iodoacetyl)-5,11-dihydro-6H-pyrido[2,3-b][1,4]benzodiazepin-6-one or a salt thereof and (R)-2-(diethylamino)methylpiperidine

or a salt thereof in an organic solvent under a basic condition.

Examples of the "organic solvent" in the method include acetonitrile, dimethylformamide (DMF), dimethylacetamide (DMA), dimethyl sulfoxide (DMSO), ethyl acetate, and others. The amount of the organic solvent added is, for example, 3 v/w to 50 v/w, preferably 5 v/w to 30 v/w, more preferably 7 v/w to 20 v/w, furthermore preferably 8 v/w to 15 v/w, and most preferably 10 v/w.

The term "v/w" in the amount of the solvent added means the volume (mL) of the organic solvent relative to the mass (1 g) of 11-(2-iodoacetyl)-5,11-dihydro-6H-pyrido[2,3-b][1,4]benzodiazepin-6-one.

Examples of the "basic condition" in the method include basic conditions with sodium carbonate, cesium carbonate, potassium carbonate, sodium hydrogen carbonate, potassium hydrogen carbonate, sodium hydride, potassium hydride, sodium hydroxide, potassium hydroxide, sodium *tert*-butoxide, potassium *tert*-butoxide, alkyllithium (e.g., normal-butyllithium, *sec*-butyllithium, *tert*-butyllithium, normal-hexyllithium), lithium amide (e.g., lithium diisopropylamide, lithium hexamethyldisilazide), sodium methoxide, *tert*-amine (e.g., trimethylamine, triethylamine, triisopropylamine, diisopropylethylamine), or others. It is preferably the basic condition with sodium hydrogen carbonate, triethylamine or diisopropylethylamine. The base may be used alone or two or more of the bases may be used in any combination and ratio. The amount of the base added is 1 to 20 equivalents, preferably 1.5 to 15 equivalents, more preferably, 2 to 10 equivalents, furthermore preferably 2.5 to 8 equivalents, particularly preferably 3 to 5 equivalents, and most preferably 3.15 equivalents.

The term "equivalent" in the amount of the base added means the amount (mol) of a base relative to the amount (1 mol) of 11-(2-iodoacetyl)-5,11-dihydro-6H-pyrido[2,3-b][1,4]benzodiazepin-6-one.

The reaction temperature in the method is, for example, 40 to 100 °C, preferably 50 to 90 °C, more preferably 60 to 80 °C, furthermore preferably 65 to 78 °C, and most preferably 70 to 75 °C.

The reaction time in the method is not particularly limited because it varies depending on various conditions such as reaction temperature. The reaction time is appropriately selected from, for example, 1 to 48 hours. The reaction time is preferably 3 to 36 hours, more preferably 6 to 30 hours, furthermore preferably 12 to 27 hours, particularly preferably 18 to 24 hours, and most preferably 20 hours.

The present invention provides a method of purifying (R)-11-[2-[2-[(diethylamino)methyl]-1-piperidinyl]acetyl]-5,11-dihydro-6H-pyrido[2,3-b][1,4]benzodiazepin-6-one or a salt thereof in high purity, which comprises crystallizing (R)-11-[2-[2-[(diethylamino)methyl]-1-piperidinyl]acetyl]-5,11-dihydro-6H-pyrido[2,3-b][1,4]benzodiazepin-6-one or a salt thereof obtained as a crude product in the preparation method of present invention in a mixture of 1-propanol and water. In the purification method, (R)-11-[2-[2-[(diethylamino)methyl]-1-piperidinyl]acetyl]-5,11-dihydro-6H-pyrido[2,3-b][1,4]benzodiazepin-6-one or a salt thereof can be crystallized by mixing (R)-11-[2-[2-[(diethylamino)methyl]-1-piperidinyl]acetyl]-5,11-dihydro-6H-pyrido[2,3-b][1,4]benzodiazepin-6-one or a salt thereof in a crude product and 1-propanol, heating and stirring the mixture, cooling, adding water thereto, stirring, and then further cooling the mixture. (R)-11-[2-[2-[(Diethylamino)methyl]-1-piperidinyl]acetyl]-5,11-dihydro-6H-pyrido[2,3-b][1,4]benzodiazepin-6-one or a salt in high purity can be obtained by aging the precipitated crystal, removing the crystal by filtration, washing, and drying the filtrate.

The amount of 1-propanol added in the purification method is, for example, 1 to 10 v/w, preferably 2 to 8 v/w, more preferably 3 to 15 v/w, and most preferably 4 v/w.

The amount of water added in the purification method is, for example, 1 to 15 v/w, preferably 2 to 10 v/w, more preferably 4 to 8 v/w, and most preferably 6 v/w.

The term "v/w" in the amounts of 1-propanol and water added means the amounts (mL) of 1-propanol and water relative to the mass (1 g) of (R)-11-[2-[2-[(diethylamino)methyl]-1-piperidinyl]acetyl]-5,11-dihydro-6H-pyrido[2,3-b][1,4]benzodiazepin-6-one or a salt thereof, respectively.

The temperature in each process of the purification method may be appropriately adjusted. For example, in the process of stirring in 1-propanol, the temperature is heated to, for example, 80 to 100 °C, preferably 85 to 95 °C. The temperature is then cooled to 40 to 60 °C, preferably 45 to 55 °C, and the addition of water and stirring are performed. The temperature is then further cooled to -5 to 10 °C, preferably 0 to 5 °C, and aging may be performed. The drying temperature in each process is not limited as long as it is the temperature range which can remove adhered moisture or solution. The drying temperature is, for example, 80 °C or less, preferably about 50 °C.

The time when stirring in 1-propanol in the purification method is not particularly limited. The time is appropriately selected from, for example, 1 to 5 hours. The time is preferably 2 to 4 hours, and most preferably 3 hours. The stirring time after the addition of water is appropriately selected from, for example, 10 minutes to 5 hours. The stirring time is preferably 20 minutes to 4 hours, and most preferably 30 minutes to 3 hours.

In the purification method, specific impurities derived from (R)-11-[2-[2-[(diethylamino)methyl]-1-piperidinyl]acetyl]-5,11-dihydro-6H-pyrido[2,3-b][1,4]benzodiazepin-6-one or a salt thereof can be removed.

The present invention also provides a method of purifying (R)-11-[2-[2-[(diethylamino)methyl]-1-piperidinyl]acetyl]-5,11-dihydro-6H-pyrido[2,3-b][1,4]benzodiazepin-6-one or a salt thereof in high purity, which comprises recrystallizing (R)-11-[2-[2-[(diethylamino)methyl]-1-piperidinyl]acetyl]-5,11-dihydro-6H-pyrido[2,3-b][1,4]benzodiazepin-6-one or a salt thereof obtained as a crude product in the preparation method of the present invention in a solvent. The purification method comprises treating (R)-11-[2-[2-[(diethylamino)methyl]-1-piperidinyl]acetyl]-5,11-dihydro-6H-pyrido [2,3-b][1,4]benzodiazepin-6-one or a salt thereof with activated carbon in an alcohol solvent, and/or recrystallizing the treated compound with a mixture of an alcohol solvent and a ketone solvent. Specifically, the treatment process with activated carbon comprises mixing AFDX0250 and activated carbon with an alcohol solvent that is good solvent, heating and stirring the mixture, filtrating the mixture through the activated carbon, and concentrating the filtrate. The recrystallization process comprises adding a ketone solvent that is poor solvent to the concentrated product, heating and stirring the mixture and cooling to crystallize AFDX0250, aging the precipitated crystal, removing the crystal by filtration, washing, and drying the filtrate. AFDX0250 in high purity can be obtained by the recrystallization process. Also, the same operations excluding the addition of activated carbon and filtration through the activated carbon may be repeated multiple times to obtain AFDX0250 in higher purity. In addition, only the recrystallization process may be performed without the treatment process with activated carbon.

Examples of the "alcohol solvent" in the purification method include methanol, ethanol, 1-propanol, 2-propanol, 1-butanol, 2-butanol, and others. It is preferably ethanol and 1-propanol. The amount of the alcohol solvent added is, for example, 5 to 50 v/w, preferably 10 to 40 v/w, more preferably 15 to 30 v/w, and most preferably 20 v/w.

The term "v/w" in the amount of the alcohol solvent added means the volume (mL) of the alcohol solvent relative to the mass (1 g) of (R)-11-[2-[2-[(diethylamino)methyl]-1-piperidinyl]acetyl]-5,11-dihydro-6H-pyrido[2,3-b][1,4]benzodiazepin-6-one or a salt thereof.

Examples of the "activated carbon" in the purification method include Shirasagi A, Shirasagi P, and others. It is preferably Shirasagi A. The amount of the activated carbon added is, for example, 0.01 to 0.3 w/w, preferably 0.03 to 0.2w/w, and most preferably 0.05 to 0.15 w/w.

The term "w/w" in the amount of the activated carbon added means the mass (g) of the activated carbon relative to the mass (1 g) of (R)-11-[2-[2-[(diethylamino)methyl]-1-piperidinyl]acetyl]-5,11-dihydro-6H-pyrido[2,3-b][1,4]benzodiazepin-6-one or a salt thereof.

In the purification method, the mixture is concentrated after the filtration through activated carbon so that the alcohol solvent is, for example, 0.5 to 3 v/w, preferably 1 to 3 v/w, more preferably 2 to 2.5 v/w. During or after the concentration of the filtrate, the ketone solvent is added in an amount of, for example, 3 to 15 v/w, preferably 5 to 10 v/w, more preferably 7 to 8 v/w to prepare a mixture of the alcohol solvent and the ketone solvent. When only the recrystallization process is performed without the treatment process with activated carbon, a mixture of an alcohol solvent and a ketone solvent is similarly used as the solvent for recrystallization. The amount of the alcohol solvent added is, for example, 0.5 to 3 v/w, preferably 1 to 3 v/w, more preferably 2 to 2.5 v/w, and the amount of the ketone solvent added is, for example, 3 to 15 v/w, preferably 5 to 10 v/w, more preferably 7 to 8 v/w.

The term "v/w" in the amount of the alcohol solvent in the mixture and the amounts of the alcohol solvent and the ketone solvent added means the volume (mL) of the mixture, the alcohol solvent or the ketone solvent relative to the mass (1 g) of (R)-11-[2-[2-[(diethylamino)methyl]-1-piperidinyl]acetyl]-5,11-dihydro-6H-pyrido[2,3-b][1,4]benzodiazepin-6-one or a salt thereof, respectively.

Examples of the "ketone solvent" in the purification method include acetone, methyl ethyl ketone (MEK), methyl isobutyl ketone (MIBK), diethyl ketone, and others. It is preferably acetone, MEK and MIBK.

The temperature in each process in the purification method may be appropriately adjusted. For example, the treatment process with activated carbon may be performed by heating and stirring the mixture at a temperature of, for example, 50 to 90 °C, preferably 60 to 80 °C, and more preferably 65 to 75 °C. The recrystallization process may be performed by crystallization at a temperature of, for example, 50 to 80 °C, preferably 60 to 70 °C, more preferably 63 to 67 °C, followed by stirring as appropriate and cooling to, for example, -10 to 15 °C, preferably -5 to 10 °C, more preferably 0 to 5 °C. Also, the aging in the recrystallization process may be performed at a temperature of, for example, -10 to 15 °C, preferably -5 to 10 °C, more preferably 0 to 5 °C.

The time when heating and stirring in the treatment process with activated carbon in the purification method is not particularly limited because it varies depending on various conditions. The time is appropriately selected from, for example, 30 minutes to 24 hours. The time is preferably 40 minutes to 12 hours, more preferably 50 minutes to 6 hours, furthermore preferably 1 to 3 hours, and most preferably 1 hour. The time when aging in the recrystallization process is, for example, 6 to 48 hours, preferably 12 to 36 hours, and most preferably 24 hours.

In the present invention, the starting material, intermediate and/or prepared compound (AFDX0250) may be in a salt form. The preparation method of the present invention also encompasses preparation methods related to their salt forms. The salts of the compounds are not particularly limited as long as they are pharmaceutically acceptable salts. Examples of the salts include salts with an inorganic acid such as hydrochloric acid, hydrobromic acid, hydroiodic acid, nitric acid, sulfuric acid, phosphoric acid; salts with an organic acid such as acetic acid, oxalic acid, fumaric acid, maleic acid, succinic acid, malic acid, citric acid, tartaric acid, adipic acid, gluconic acid, glucoheptonic acid, glucuronic acid, terephthalic acid, methanesulfonic acid, alanine, lactic acid, hippuric acid, 1,2-ethanedisulfonic acid, isethionic acid, lactobionic acid, oleic acid, gallic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid, sulfosalicylic acid; salts with a metal such as sodium, potassium, calcium, magnesium; salts with an inorganic compound such as ammonia; and salts with an organic amine such as triethylamine, guanidine.

In the present invention, the starting material, intermediate and/or prepared compound (AFDX0250) or salts thereof may be in the form of a hydrate or a solvate.

According to the preparation method and purification method of the present invention, (R)-11-[2-[2-[(diethylamino)methyl]-1-piperidinyl]acetyl]-5,11-dihydro-6H-pyrido[2,3-b][1,4]benzodiazepin-6-one or a salt thereof in high purity can be obtained. The purity of (R)-11-[2-[2-[(diethylamino)methyl]-1-piperidinyl]acetyl]-5,11-dihydro-6H-pyrido[2,3-b][1,4]benzodiazepin-6-one or a salt thereof is basically 95% or more, preferably 97% or more, more preferably 99% or more, and most preferably 99.5% or more.

According to the preparation method and purification method of the present invention, the amount of impurities in (R)-11-[2-[2-[(diethylamino)methyl]-1-piperidinyl]acetyl]-5,11-dihydro-6H-pyrido[2,3-b][1,4]benzodiazepin-6-one or a salt thereof can be reduced to, for example, less than 5%, preferably less than 3%, more preferably 1%, and most preferably 0.5%. The "impurities" as used herein are impurities derived from (R)-11-[2-[2-[(diethylamino)methyl]-1-piperidinyl]acetyl]-5,11-dihydro-6H-pyrido[2,3-b][1,4]benzodiazepin-6-one or a salt thereof and means 11-(2-chloroacetyl)-5,11-dihydro-6H-pyrido[2,3-b][1,4]benzodiazepin-6-one, 5,11-dihydro-6H-pyrido[2,3-b][1,4]benzodiazepin-6-one, 11-(2-iodoacetyl)-5,11-dihydro-6H-pyrido[2,3-b][1,4]benzodiazepin-6-one and other substances contaminated during the preparation or purification of (R)-11-[2-[2-[(diethylamino)methyl]-1-piperidinyl]acetyl]-5,11-dihydro-6H-pyrido[2,3-b][1,4]benzodiazepin-6-one or a salt thereof.

In the present invention, the purity of (R)-11-[2-[2-[(diethylamino)methyl]-1-piperidinyl]acetyl]-5,11-dihydro-6H-pyrido[2,3-b][1,4]benzodiazepin-6-one or a salt thereof and the amount of impurities therein may be measured by quantifying the peak area ratio in liquid chromatography (HPLC), which is the well-known method in the analytical chemistry, by the area normalization method and quantifying by liquid chromatography mass spectrometry (LC/MS).

(1) Measurement method of amount of (R)-11-[2-[2-[(diethylamino)methyl]-1-piperidinyl]acetyl]-5,11-dihydro-6H-pyrido[2,3-b][1,4]benzodiazepin-6-one (AFDX0250) or a salt thereof

The amount of AFDX0250 or a salt thereof can be measured by, for example, the following procedure.

<Mobile phase and Eluent>

The following mobile phases and eluents are used.

- · Mobile phase A: 0.1 mol/L sodium perchlorate solution (pH 3.0)
  Sodium perchlorate monohydrate (14.0 g) is dissolved in water (1000 mL) and 1 mol/L hydrochloric acid sample solution is added to adjust the pH of the solution to 3.0.
- · Mobile phase B: Acetonitrile
- · Eluent A: Mobile phase A : Mobile phase B (1:1) (v/v)

<Preparation of AFDX0250 standard solution>

AFDX250 (about 10 mg) is accurately weighed and Eluent A is added to adjust the solution exactly to 100 mL.

<Preparation of sample solution>

Sample solutions are prepared by the following procedure.

- · Sample solution A: The reaction suspension comprising AFDX0250 (reaction solution obtained in Step 1 of Example 1 below) (100 to 200 mg) is taken and dissolved in Eluent A (10 mL).
- · Sample solution B: The reaction suspension comprising AFDX0250 (reaction solution obtained in Step 1 of Example 1 below) (about 200 μL) is taken and filtrated *in vacuo* using Kiriyama funnel (S-40), Kiriyama filter paper (No. 3) and filtering flask, and the resulting filtrate (about 10 mg) is dissolved in Eluent A (10 mL).
- ·Sample solution C: The obtained solid (AFDX0250 obtained in Step 3 of Example 1 below) (about 10 mg) is accurately taken and Eluent A is added to adjust the solution exactly to 100 mL.
- · Sample solution D: The aqueous layer (aqueous layer obtained in Step 2 of Example 1 below) (about 100 μL) is accurately taken and Eluent A is added to adjust the solution exactly to 10 mL.

<Measurement method>

Each sample solution is measured by liquid chromatography based on the following procedure and condition. In addition, the amount of AFDX0250 (%) is calculated according to the following calculation formula.

(Test condition of liquid chromatography (UHPLC))

· Detector: UV absorption spectrophotometer (Measurement Wavelength 210nm)
· Column: AQUITY UPLC HSS T3 (Inner Diameter 2.1 mm, Length 100 mm, Particle Size 1.8 μm; Waters)
· Ghost Trap: Ghost Trap DS-HP (P/N: 228-59931-91) (Shimadzu Corporation) or Ghost Trap with equivalent performance quality (Ghost Trap is connected after mobile phase mixer in order to remove impurities in mobile phase.)
· Column Temperature: About 40 °C
· Sample Cooler Temperature: About 10 °C
· Mobile phase A: 0.1 mol/L sodium perchlorate solution (pH 3.0)
·Mobile phase B: Acetonitrile
· Liquid delivery of mobile phase: The mixing ratio of Mobile phase A and Mobile phase B is controlled by concentration gradient as shown in Table 1.

[Table 1]

| Time from sample injection (min) | Mobile phase A (%) | Mobile phase B (%) |
|---|---|---|
| 0.00 - 16.00 | 95 → 40 | 5 → 60 |
| 16.00 - 20.00 | 40 | 60 |
| 20.00 - 20.10 | 40 → 95 | 60 → 5 |
| 20.10 - 24.00 | 95 | 5 |

· Flow rate: 0.5 mL/min
·Injection volume: 2 μL
·Area measurement range: From the peak of the solvent to 20 minutes after injection

(Calculation Formula)

Sample solution A to C:

$$\text{Amount of AFDX0250 (\%)} = 100 \text{ x } AAp/ATp$$

AAp: Peak area of AFDX0250 obtained from each sample solution
ATp: Total peak area obtained from each sample solution

Sample solution D:

$$\text{Amount of AFDX0250 (\%)} = 100 \text{ x } MS \text{ x } AT \text{ x } 10/(MT \text{ x } AS \text{ x } 100)$$

MS: Collected amount of AFDX0250 (mg)
MT: Collected amount of sample solution (mg)
AS: AFDX0250 peak area obtained from standard solution
AT: AFDX0250 peak area obtained from sample solution

(2) Measurement method of amount of 11-(2-chloroacetyl)-5,11-dihydro-6H-pyrido[2,3-b][1,4]benzodiazepin-6-one (Compound 1)

The amount of Compound 1 contained in (R)-11-[2-[2-[(diethylamino)methyl]-1-piperidinyl]acetyl]-5,11-dihydro-6H-pyrido[2,3-b][1,4]benzodiazepin-6-one or a salt thereof can be measured by, for example, the following procedure.

<Mobile phase and Eluent>

The following mobile phases and eluent are used.

- Mobile phase C: 10 mM ammonium acetate solution

Ammonium acetate (0.77 g) is dissolved in water (1000 mL).

· Mobile phase B: Acetonitrile
· Eluent B: Mobile phase C : Mobile phase B (7:3) (v/v)

<Preparation of Compound 1 standard solution>

· Compound 1 standard undiluted solution: Compound 1 (about 20 mg) is accurately weighed and Eluent B is added to adjust the solution exactly to 100 mL. When the compound is difficult to dissolve, it is dissolved using ultrasonic waves for about 1 minute. The solution (2 mL) is accurately taken and Eluent B is added to adjust the solution exactly to 20 mL. The solution (1 mL) is accurately taken and Eluent B is added to adjust the solution exactly to 20 mL.
· Compound 1 standard solution 1: The standard undiluted solution (4 mL) is accurately taken and Eluent B is added to adjust the solution exactly to 20 mL.
· Compound 1 standard solution 2: The standard solution 1 (2 mL) is accurately taken and Eluent B is added to adjust the solution exactly to 10 mL.
· Compound 1 standard solution 3: The standard solution 2 (2 mL) is accurately taken and Eluent B is added to adjust the solution exactly to 20 mL.

<Preparation of sample solution>

Sample solutions are prepared by the following procedure.

· Sample solution E (when measurement target is aqueous layer): The aqueous layer obtained by the above (1) Measurement method is accurately taken so that AFDX0250 contained is about 100 mg calculated from the amount of AFDX0250 (%) in the aqueous layer is accurately taken and Eluent B is added to adjust the solution exactly to 25 mL.
· Sample solution F (when measurement target is crystal): AFDX0250 (about 100 mg) is accurately weighed and dissolved in Eluent B (5 mL) and diluted hydrochloric acid (0.2 mL), and Eluent B is added to adjust the solution exactly to 25 mL (AFDX0250 concentration: about 4 mg/mL). When the compound is difficult to dissolve, it is dissolved using ultrasonic waves.

<Measurement method>

Each sample solution is measured by liquid chromatography mass spectrometry based on the following condition in the order and number of measurements shown in Table 3 below. In addition, calibration curves are created from the concentrations (μg/mL) and the peak areas of standard solutions (using the total 6 results of standard solutions 1 to 3), and the concentration of Compound 1 (μg/mL) in each sample solution and the amount of Compound 1 (ppm) in AFDX0250 are calculated according to the following calculation formula.

(Test condition of liquid chromatography mass spectrometry (LC/MS))

· Detector: Mass Spectrometer
· Monitor Ion: m/z 288 (ESI, Positive)

· Column: X Bridge C18 (Inner Diameter 2.1 mm, Length 150 mm, Particle Size 3.5 μm; Waters)
· Column Temperature: About 40 °C
· Sample Cooler Temperature: Temperature without artificial control
· Mobile phase C: 10 mM ammonium acetate solution
· Mobile phase B: Acetonitrile
· Liquid delivery of mobile phase: The mixing ratio of Mobile phase C and Mobile phase B is controlled by concentration gradient as shown in Table 2.

[Table 2]

| Time from sample injection (min) | Mobile phase A (%) | Mobile phase B (%) |
|---|---|---|
| 0 - 40.0 | 95 → 40 | 5 → 60 |
| 40.0 - 40.5 | 40 → 5 | 60 → 95 |
| 40.5 - 45.0 | 5 | 95 |
| 45.0 - 45.5 | 5 → 95 | 95 → 5 |
| 45.5 - 55.5 | 95 | 5 |

· Flow rate: 0.3 mL/min
· Injection volume: 5 μL
· Order and number of measurements of sample solution:

[Table 3]

| Order of Measurement | Sample Solution | Number of measurements, etc. |
|---|---|---|
| 1 | Blank solution (Eluent B) | Once |
| 2 | Standard solution 3 | Twice: Creation of calibration curve |
| 3 | Standard solution 2 | Twice: Creation of calibration curve |
| 4 | Standard solution 1 | Twice: Creation of calibration curve |
| 5 | Blank solution (Eluent B) | Once |
| 6 | Sample solution E or Sample solution F | Once |

(Calculation Formula)

<u>Sample solution E (when measurement target is aqueous layer):</u>

Concentration of Compound 1 in Sample solution E (μg/mL) = (AT - b)/a

Amount of Compound 1 in AFDX0250 (ppm) = Concentration of Compound 1 in Sample solution E (μg/mL)/MT × P/100 × 25000

AT: Peak Area of Compound 1 obtained from Sample solution E
b: Intercept of calibration curve
a: Slope of calibration curve
MT: Amount of weighed sample (mg)
P: Amount of AFDX0250 (%) in aqueous layer obtained according to the above (1) Measurement method

<u>Sample solution F (when measurement target is crystal):</u>

Concentration of Compound 1 in Sample solution F (μg/mL) = (AT-b)/a

Amount of Compound 1 in AFDX0250 (ppm) = Concentration of Compound 1 in Sample solution F (μg/mL)/MT × 25000

AT: Peak Area of Compound 1 obtained from Sample solution F
b: Intercept of calibration curve
a: Slope of calibration curve
MT: Amount of weighed sample (mg)

The high purity of (R)-11-[2-[2-[(diethylamino)methyl]-1-piperidinyl]acetyl]-5,11-dihydro-6H-pyrido[2,3-b][1,4]benzodiazepin-6-one of the present invention has higher purity of 95% or more and contains smaller amounts of impurities than (R)-11-[2-[2-[(diethylamino)methyl]-1-piperidinyl]acetyl]-5,11-dihydro-6H-pyrido[2,3-b][1,4]benzodiazepin-6-one prepared by the conventional known methods, and thus it is expected that it is used as pharmaceutical products with higher safety.

The present invention also provides a pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the pharmaceutical composition is substantially free of impurities.

The pharmaceutical composition of the present invention comprises (R)-11-[2-[2-[(diethylamino)methyl]-1-piperidinyl]acetyl]-5,11-dihydro-6H-pyrido[2,3-b][1,4]benzodiazepin-6-one or a salt thereof with a purity of 95% or more, preferably a purity of 97% or more, more preferably a purity of 99% or more, and most preferably a purity of 99.5% or more.

The pharmaceutical composition of the present invention preferably is substantially free of 11-(2-chloroacetyl)-5,11-dihydro-6H-pyrido[2,3-b][1,4]benzodiazepin-6-one or a salt thereof, more preferably is substantially free of further 5,11-dihydro-6H-pyrido[2,3-b][1,4]benzodiazepin-6-one or a salt thereof, and particularly preferably is substantially free of not only 11-(2-chloroacetyl)-5,11-dihydro-6H-pyrido[2,3-b][1,4]benzodiazepin-6-one and 5,11-dihydro-6H-pyrido[2,3-b][1,4]benzodiazepin-6-one but also substances contaminated during the preparation or purification of (R)-11-[2-[2-[(diethylamino)methyl]-1-piperidinyl]acetyl]-5,11-dihydro-6H-pyrido[2,3-b][1,4]benzodiazepin-6-one or a salt thereof. 5,11-Dihydro-6H-pyrido[2,3-b][1,4]benzodiazepin-6-one is a compound represented by the following structural formula:

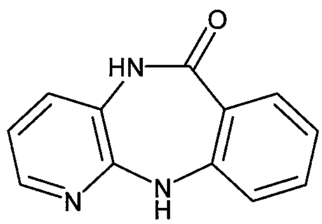

The term "substantially free of" as used herein means that no impurity such as 11-(2-chloroacetyl)-5,11-dihydro-6H-pyrido[2,3-b][1,4]benzodiazepin-6-one is contained, that the presence of impurities cannot be detected by commonly-used methods for the analysis of pharmaceutical products such as liquid column chromatography using chiral column (Detector: UV absorption spectrophotometer), or that impurities are contained in an amount such that they do not produce their effects and side effects. For example, the amounts of impurities in the pharmaceutical composition is less than 5%, preferably less than 3%, more preferably less than 1%, and most preferably less than 0.5%, relative to (R)-11-[2-[2-[(diethylamino)methyl]-1-piperidinyl]acetyl]-5,11-dihydro-6H-pyrido[2,3-b][1,4]benzodiazepin-6-one or a salt thereof.

In the present invention, the amount of 11-(2-chloroacetyl)-5,11-dihydro-6H-pyrido[2,3-b][1,4]benzodiazepin-6-one or a salt thereof in the pharmaceutical composition is, for example, less than 1000 ppm, preferably less than 700 ppm, more preferably less than 500 ppm, furthermore preferably less than 300 ppm, even more preferably less than 100 ppm, particularly preferably less than 10 ppm, and most preferably less than 2 ppm, relative to (R)-11-[2-[2-[(diethylamino)methyl]-1-piperidinyl]acetyl]-5,11-dihydro-6H-pyrido[2,3-b][1,4]benzodiazepin-6-one or a salt thereof.

In the present invention, the amount of 5,11-dihydro-6H-pyrido[2,3-b][1,4]benzodiazepin-6-one or a salt thereof in the pharmaceutical composition is, for example, less than 1000 ppm, more preferably less than 800 ppm, more preferably less than 600 ppm, and most preferably less than 500 ppm, relative to (R)-11-[2-[2-[(diethylamino)methyl]-1-piperidinyl]acetyl]-5,11-dihydro-6H-pyrido[2,3-b][1,4]benzodiazepin-6-one or a salt thereof.

In the present invention, the total amount of 11-(2-chloroacetyl)-5,11-dihydro-6H-pyrido[2,3-b][1,4]benzodiazepin-6-one and 5,11-dihydro-6H-pyrido[2,3-b][1,4]benzodiazepin-6-one in the pharmaceutical composition is, for example, less than 2000 ppm, preferably less than 1000 ppm, more preferably less than 700 ppm, and most preferably less

than 500 ppm, relative to (R)-11-[2-[2-[(diethylamino)methyl]-1-piperidinyl]acetyl]-5,11-dihydro-6H-pyrido[2,3-b][1,4]benzodiazepin-6-one or a salt thereof.

The pharmaceutical composition of the present invention may comprise a pharmaceutically acceptable active ingredient other than (R)-11-[2-[2-[(diethylamino)methyl]-1-piperidinyl]acetyl]-5,11-dihydro-6H-pyrido[2,3-b][1,4]benzodiazepin-6-one or a salt thereof unless otherwise specified. In one embodiment, the pharmaceutical composition of the present invention comprises no pharmaceutically acceptable active ingredient other than (R)-11-[2-[2-[(diethylamino)methyl]-1-piperidinyl]acetyl]-5,11-dihydro-6H-pyrido[2,3-b][1,4]benzodiazepin-6-one or a salt thereof.

The pharmaceutical composition of the present invention may comprise an additive as appropriate. Examples of the additive include a surfactant, a tonicity agent, a stabilizing agent, a preservative, an antioxidant, a high molecular weight polymer, a pH adjuster, and a base.

The pharmaceutical composition of the present invention is preferably an aqueous pharmaceutical composition, but may be a non-aqueous pharmaceutical composition.

The pharmaceutical composition of the present invention may be administered orally or parenterally. Examples of the administration route include oral administration, intravenous administration, transdermal administration, ocular topical administration (e.g., instillation administration, conjunctival sac administration, intravitreal administration, subconjunctival administration, sub-Tenon's administration), and others.

The dosage form of the pharmaceutical composition of the present invention is not particularly limited as long as it can be used as a pharmaceutical product. Examples thereof include an eye drop, an eye gel, an injection, and others. The pharmaceutical composition of the present invention is particularly preferably used as an eye drop. Also, when the pharmaceutical composition of the present invention is used as a non-aqueous pharmaceutical composition, it may be formulated as a non-aqueous preparation.

They may be prepared according to any commonly-used methods in the art.

When the pharmaceutical composition of the present is prepared as an eye drop, a desired eye drop can be prepared by adding an active ingredient into a medium such as purified water and buffer solution and stirring, and then adjusting the pH of the solution with a pH adjuster.

Also, an additive which is commonly-used in an eye drop may be used as appropriate. Examples of the additive include an isotonic agent, a buffering agent, a surfactant, a stabilizing agent, a preservative, a solubilizing agent, and others.

When the pharmaceutical composition of the present invention is prepared as an oral agent such as tablet, capsule, granule and powder, it may be prepared by adding an additive such as a bulking agent, a lubricant, a binding agent, a disintegrant, a coating agent, a filming agent, and others to the pharmaceutical composition as appropriate. Examples of the bulking agent include lactose, crystalline cellulose, starch, vegetable oil, and others, examples of the lubricant include magnesium stearate, talc, and others, examples of the binding agent include hydroxypropyl cellulose, polyvinylpyrrolidone, and others, examples of the disintegrant include calcium carboxymethylcellulose, lowsubstituted hydroxypropyl methylcellulose, and others, examples of the coating agent include hydroxypropyl methylcellulose, macrogol (polyethylene glycol), silicone resin, and others, and examples of the filming agent include gelatin film and others.

The dose and usage of the pharmaceutical composition of the present invention may be appropriately varied according to various factors such as the dosage form, the symptom, age, body weight or age at onset of disease of a patient, and physician's decision. For example, when the pharmaceutical composition of the present invention is ocular-topically administered as an eye drop, the dose and usage thereof are not particularly limited as long as they are sufficient to produce the desired efficacy. It may be administered to eyes at a dose of one drop to several drops per time once to several times (e.g., once to four times, once to six times, once to eight times) daily. Also, the pharmaceutical composition of the present invention may be used when wearing contact lenses.

The pharmaceutical composition of the present invention may be used for the treatment of an eye disease such as glaucoma, ocular hypertension, and myopia.

## EXAMPLES

Hereinafter, the present invention is illustrated in Examples in order to make it easy to understand the present invention. The present invention, however, is not intended to be limited thereto by any means.

In the following examples, HPLC (UHPLC) analysis and LC/MS analysis are performed under the following condition unless otherwise specified.

(HPLC (UHPLC) analysis condition)

·Detector: UV absorption spectrophotometer (Measurement Wavelength 210nm)

·Column: AQUITY UPLC HSS T3 (Inner Diameter 2.1 mm, Length 100 mm, Particle Size 1.8 μm; Waters)
·Ghost Trap: Ghost Trap DS-HP (P/N: 228-59931-91) (Shimadzu Corporation) or Ghost Trap with equivalent performance quality (Ghost Trap is connected after mobile phase mixer in order to remove impurities in mobile phase.)
· Column Temperature: About 40 °C
· Sample Cooler Temperature: About 10 °C
· Mobile phase A: 0.1 mol/L sodium perchlorate solution (pH 3.0)
· Mobile phase B: Acetonitrile
· Liquid delivery of mobile phase: The mixing ratio of Mobile phase A and Mobile phase B is controlled by concentration gradient as shown in Table 4.

[Table 4]

| Time from sample injection (min) | Mobile phase A (%) | Mobile phase B (%) |
|---|---|---|
| 0.00 - 16.00 | 95 → 40 | 5 → 60 |
| 16.00 - 20.00 | 40 | 60 |
| 20.00 - 20.10 | 40 → 95 | 60 → 5 |
| 20.10 - 24.00 | 95 | 5 |

·Flow rate: 0.5 mL/min
·Injection volume: 2 μL
·Area measurement range: From the peak of the solvent to 20 minutes after injection

(LC/MS analysis condition)

· Detector: Mass Spectrometer
· Monitor Ion: m/z 288 (ESI, Positive)
· Column: X Bridge C18 (Inner Diameter 2.1 mm, Length 150 mm, Particle Size 3.5 μm; Waters)
· Column Temperature: About 40 °C
· Sample Cooler Temperature: Ambient temperature ·Mobile phase C: 10 mM ammonium acetate solution
· Mobile phase B: Acetonitrile
· Liquid delivery of mobile phase: The mixing ratio of Mobile phase C and Mobile phase B is controlled by concentration gradient as shown in Table 5.

[Table 5]

| Time from sample injection (min) | Mobile phase A (%) | Mobile phase B (%) |
|---|---|---|
| 0 - 40.0 | 95 → 40 | 5 → 60 |
| 40.0 - 40.5 | 40 → 5 | 60 → 95 |
| 40.5 - 45.0 | 5 | 95 |
| 45.0 - 45.5 | 5 → 95 | 95 → 5 |
| 45.5 - 55.5 | 95 | 5 |

· Flow rate: 0.3 mL/min
· Injection volume: 5 μL
· Order and number of measurements of sample solution:

[Table 6]

| Order of Measurement | Sample Solution | Number of measurements, etc. |
|---|---|---|
| 1 | Blank solution (the above Eluent B) | Once |
| 2 | Standard solution 3 (the above Compound 1 standard solution 3) | Twice: Creation of calibration curve |

(continued)

| Order of Measurement | Sample Solution | Number of measurements, etc. |
|---|---|---|
| 3 | Standard solution 2 (the above Compound 1 standard solution 2) | Twice: Creation of calibration curve |
| 4 | Standard solution 1 (the above Compound 1 standard solution 1) | Twice: Creation of calibration curve |
| 5 | Blank solution (the above Eluent B) | Once |
| 6 | Sample solution | Once |

Example 1: Preparation of AFDX0250 (1)

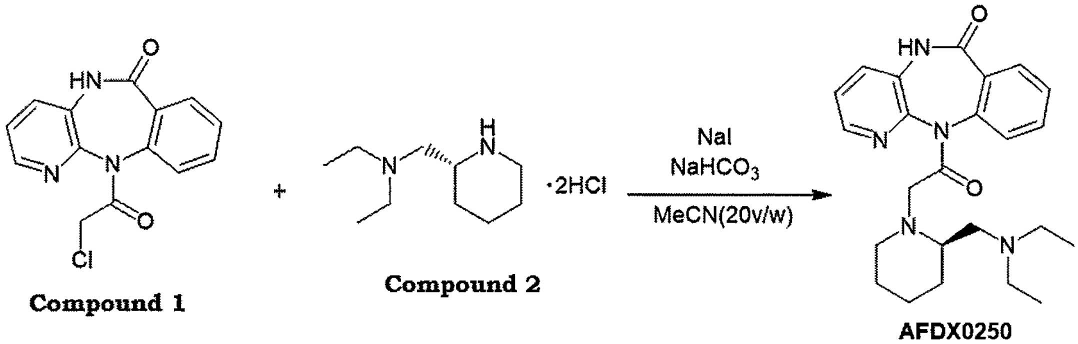


Compound 1 Compound 2 AFDX0250

According to the following steps, (R)-11-[2-[2-[(diethylamino)methyl]-1-piperidinyl]acetyl]-5,11-dihydro-6H-pyrido[2,3-b][1,4]benzodiazepin-6-one (AFDX0250) was prepared.

Step 1

To a solution of 11-(2-chloroacetyl)-5,11-dihydro-6H-pyrido[2,3-b][1,4]benzodiazepin-6-one (Compound 1) (200 g, 0.695 mol) in acetonitrile (3200 g) were added (R)-2-(diethylamino)methylpiperidine dihydrochloride (hereinafter also referred to as "Compound 2"; 178 g, 0.732 mol), sodium iodide (26.0 g, 0.174 mol) and sodium hydrogen carbonate (184 g, 2.19 mol) at room temperature under nitrogen atmosphere and heated at an internal temperature of 73.0 to 74.5°C. The reaction solution at each point (2 hours, 3 hours, 4 hours, and 5 hours after the reaction) was measured by liquid chromatography (UHPLC) to quantify AFDX0250, Compound 1 and 5,11-dihydro-6H-pyrido[2,3-b][1,4]benzodiazepin-6-one (hereinafter also referred to as "Compound 3") by the area normalization method.

Step 2

After the reaction for 5 hours in Step 1, the reaction solution was cooled and concentrated to obtain AFDX0250 (1231.56 g) as a crude product. Water (1800 g) was added thereto and concentrated hydrochloric acid (155 mL) was added dropwise at an internal temperature of 4.0 to 5.5°C until the pH was 1 or less (pH 0.8), and then the mixture was separated with ethyl acetate (1800 g) three times. The separated aqueous layer at each point was measured by liquid chromatography (UHPLC) to quantify AFDX0250 and Compound 3 by the area normalization method. Also, the amount of Compound 1 was measured by liquid chromatography mass spectrometry (LC/MS).

Step 3

To the aqueous layer obtained in Step 2 were added water (1400 g) and acetonitrile (780 g), the mixture was stirred at an internal temperature of 2.0 to 5.0°C, and then 3.25N aqueous potassium hydroxide solution was added dropwise until the pH is 11 or less, and the mixture was stirred for 5 hours. The precipitated solid was filtrated *in vacuo* and the filtrated product was washed with water (2000 g), and then the mixture was dried *in vacuo* at 50 °C to obtain crude AFDX0250 (274.35 g, Yield 93.3%) as a light brown solid. The resulting light brown solid was measured by liquid chromatography (UHPLC) to quantify AFDX0250 and Compound 3 by the area normalization method. Also, the amount of

Compound 1 was measured by liquid chromatography mass spectrometry (LC/MS).

The amounts of AFDX0250 and Compound 3 in the reaction solution obtained in Step 1, the aqueous layer obtained in Step 2 and the solid obtained in Step 3 as well as the amount of Compound 1 in the reaction solution obtained in Step 1 were measured by (1) Measurement method of amount of AFDX0250 or a salt thereof as described herein.

Also, the amount of Compound 1 contained in AFDX0250 in the aqueous layer obtained in Step 2 and the solid obtained in Step 3 was measured by (2) Measurement method of amount of Compound 1 as described herein.

The UHPLC analysis and LC/MS analysis results of the reaction solution and sample solution in each step are shown in Table 7. Table 7 shows the amounts of AFDX0250, Compound 1 and Compound 3. The "a)" in the table shows the amount of Compound 1 measured in liquid chromatography mass spectrometry.

[Table 7]

| Operation | Measurement target | AFDX0250 | Compoud 1 | Compound 3 |
|---|---|---|---|---|
| After 2hr of reaction | Slurry | 90.41% | 5.58% | 0.77% |
| | Crystal | 89.84% | 5.29% | 0.73% |
| After 3hr of reaction | Slurry | 95.26% | 0.878% | 0.90% |
| | Crystal | 98.24% | 0.504% | 0.10% |
| After 4hr of reaction | Slurry | 96.23% | 0.0986% | 0.96% |
| | Crystal | 98.76% | 0.188% | 0.09% |
| After 5hr of reaction | Slurry | 96.66% | 0.0659% | 0.95% |
| | Crystal | 98.78% | 0.129% | 0.09% |
| 1st liquid separation | Aqueous layer | 96.63% | 58.7ppm[a)] | 0.77% |
| 2nd liquid separation | Aqueous layer | 96.95% | 14.1ppm[a)] | 0.63% |
| 3rd liquid separation | Aqueous layer | 97.21 % | 4.6ppm[a)] | 0.54% |
| After drying | Acquired solid crude AFDX0250 | 98.43% | 3.1ppm[a)] | 0.44% |

In this study, it was shown that in the preparation method of the present invention, Compound 1 which was positive in Ames test could be removed to a level where it was not detected and AFDX0250 could be obtained in high yield and high purity. Hence, the preparation method of the present invention can prepare AFDX0250 in high yield and high purity.

In addition, it was shown that AFDX0250 could be prepared even when using triethylamine in place of sodium hydrogen carbonate in the above Step 1.

Example 2: Assay of AFDX0250 prepared in preparation method of the present invention and known preparation method

The assay of AFDX0250 prepared in the preparation method of the present invention (20 mL acetonitrile per g of Compound 1 was used as reaction solvent) and the known preparation method (10 mL acetonitrile per g of Compound 1 was used) was performed to compare the area % of AFDX0250 and impurities.

Specifically, AFDX0250 was prepared in the same manner as in Example 1 changing the batch amount of Compound 1 from 200 g to 95 g and using the amounts (equivalents per amount of Compound 1) of Compound 2 and reagents in the same amounts as Example 1 (Example 2). Also, AFDX0250 was prepared in the same manner as in Example 2 except that 10 mL of acetonitrile per g of Compound 1 was used in place of 20 mL of acetonitrile per g of Compound 1 (Comparative Example 1). Sample solution was prepared using the resulting reaction solution in the same manner as in Example 1. UHPLC analysis was performed for each sample solution.

The UHPLC analysis results of AFDX0250 obtained in the preparation method of the present invention (Example 2) and the known preparation method (Comparative Example 1) are shown in Table 8.

[Table 8]

| Operation | Measurement target | Comparative Example 1 | | | Example 2 | | |
|---|---|---|---|---|---|---|---|
| | | AFDX0250 | Compound 1 | Compound 3 | AFDX0250 | Compound 1 | Compound 3 |
| After 2hr of reaction | Slurry | - | - | - | 92.70% | 3.14% | 0.83% |
| | Crystal | - | - | - | 90.90% | 4.84% | 0.77% |
| After 4hr of reaction | Slurry | - | - | - | 96.05% | 0.12% | 0.97% |
| | Crystal | - | - | - | 98.80% | 0.19% | 0.07% |
| After 20hr of reaction | Slurry | 95.51% | 0.36% | 1.19% | 96.16% | 0.01% | 1.08% |
| | Crystal | 98.17% | 0.79% | 0.11% | 99.40% | 0.03% | 0.07% |
| After 24hr of reaction | Slurry | 95.31% | 0.32% | 1.29% | - | - | - |
| | Crystal | 98.33% | 0.71% | 0.10% | - | - | - |
| After 40hr of reaction | Slurry | 95.18% | 0.27% | 1.43% | - | - | - |
| | Crystal | 98.64% | 0.47% | 0.11% | - | - | - |

In this study, it was shown that in the preparation method of the present invention, impurities including Compound 1 could be significantly removed. Hence, it was shown that the preparation method of the present invention improved the purity of AFDX0250.

<u>Example 3: Preparation of AFDX0250 (2)</u>

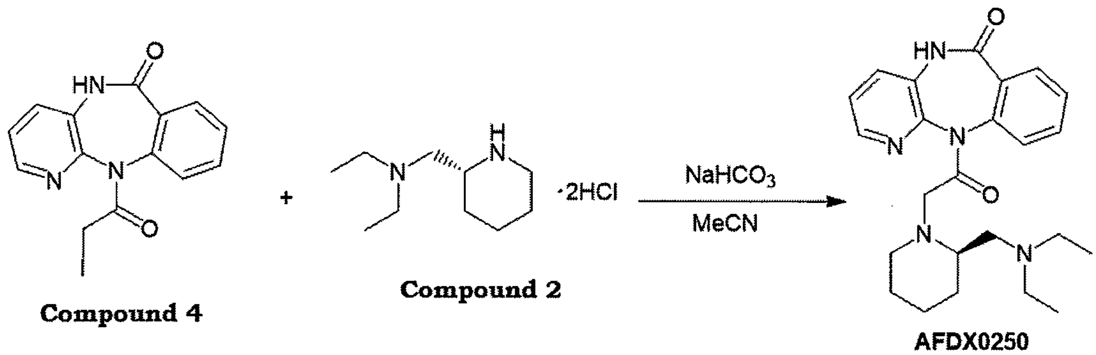


Compound 4

Compound 2

AFDX0250

To a solution of 11-(2-iodoacetyl)-5,11-dihydro-6H-pyrido[2,3-b][1,4]benzodiazepin-6-one (hereinafter also referred to as "Compound 4") (2.00 g, 5.27 mol) in acetonitrile (40.0 mL) were added Compound 2 (135 g, 5.53 mol) and sodium hydrogen carbonate (140 g, 16.6 mol) at room temperature under nitrogen atmosphere and heated at an internal temperature of 73.0 °C for 23 hours. The reaction solution was filtrated *in vacuo* and the resulting fitlrated product was rinsed with acetonitrile (2.0 mL) five times. The filtrate was concentrated to dryness, acetonitrile (7.60 mL) and water (9.20 mL) were added to the residue and stirred to dissolve, and the mixture was cooled to 0 °C. To the solution was added 3.25 N potassium hydroxide until the pH is 11.2 to 11.4 and the mixture was sitrred at 0 °C for 3 hours. The precipitated solid was filtrated *in vacuo* and rinsed with water (13.2 mL) to obtain a wet solid. The solid was dried *in vaco* at 50 °C to obtain AFDX0250 (1.04 g, Yield 46.9 %) as a light brown solid.

The $^1$ H-NMR data of AFDX0250 is as follows.

$^1$ H-NMR (400 MHz, $CDCl_3$) δ 0.83-0.97 (m, 6H), 1.07-1.80 (m, 6H), 1.95-2.63 (m, 7.5H), 2.72-2.82 (m, 0.5H), 3.07-3.27 (m, 0.5H), 3.68 (s, 1H), 4.10-4.28 (m, 0.5H), 7.27-7.33 (m, 1H), 7.36-7.48 (m, 1H), 7.55-7.70 (m, 3H), 7.93 (d, J=7.8Hz, 1H), 8.30 (d, J=2.5Hz, 1H), 9.90-10.30 (br, 1H).

In this study, it was shown that (R)-11-[2-[2-[(diethylamino)methyl]-1-piperidinyl]acetyl]-5,11-dihydro-6H-pyrido [2,3-b][1,4]benzodiazepin-6-one could be prepared even when reacting 11-(2-iodoacetyl)-5,11-dihydro-6H-pyrido[2,3-b] [1,4]benzodiazepin-6-one with (R)-2-(diethylamino)methylpiperidine dihydrochloride.

Example 4: Purification of AFDX0250 (1)

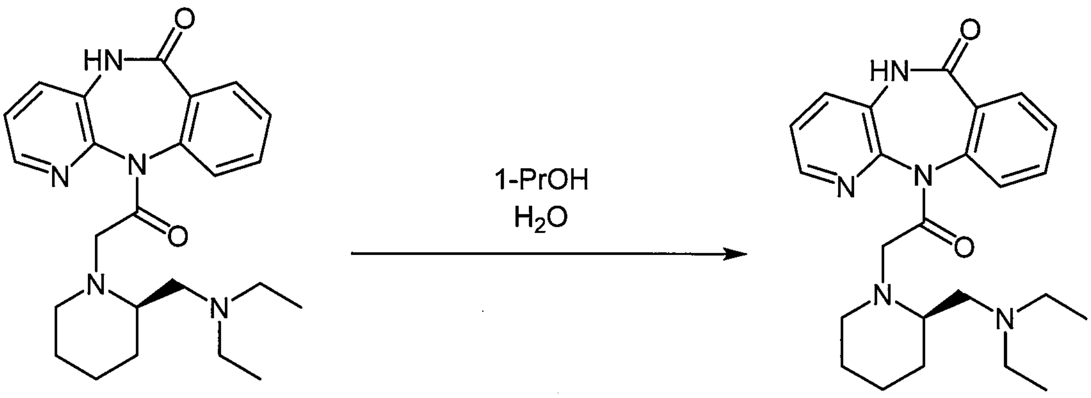


AFDX0250

AFDX0250

A solution of the crude AFDX0250 (250 g, 0.593 mol) obtained in Example 1 in 1-propanol (800 g) prepared at room temperature under nitrogen atmosphere was heated and stirred at an internal temperature of 89.7 to 90.1 °C for 3 hours, cooled to an internal temperature of 50.1 °C at a cooling rate of 0.5 °C/min, water (1500 g) was added thereto over 30 minutes at an internal temperature of 48.1 to 50.9 °C, and then the mixture was stirred at 50.3 to 50.9 °C for 30 minutes. The mixture was then cooled to an internal temperature of 6.3 °C at a cooling rate of 0.5 °C/min and stirred at an internal temperature of 0.8 to 6.3 °C for 17 hours. The precipitated solid was filtrated *in vacuo* and the filtrated product was washed with water (2500 g), and then the mixture was dried *in vacuo* at 50 °C to obtain crude AFDX0250 (217.09 g, Yield 86.8%) purified as a light brown solid. The resulting light brown solid measured by liquid chromatography (UHPLC) to quantify AFDX0250, Compound 1, Compound 3, and the compound with a relative retention time of 1.06 (hereinafter also referred to as "Compound 5") by the area normalization method. Also, the amount of Compound 1 was measured by liquid chromatography mass spectrometry (LC/MS).

The UHPLC analysis and LC/MS analysis results are shown in Table 9. The "a)" in the table shows the amount of Compound 1 measured by liquid chromatography mass spectrometry.

[Table 9]

| Measurement object | AFDX0250 | Compound 1 | Compound 3 | Compound 5 |
|---|---|---|---|---|
| Crude AFDX0250 | 98.43% | 3.1 ppm[a)] | 0.44% | 0.44% |
| Crude AFDX0250 purified | 99.47% | 1.1 ppm[a)] | 0.03% | 0.22% |

In this study, it was shown that in the purification method of the present invention, AFDX0250 could be obtained in high yield and Compound 5, which is an impurity, could be further reduced beside Compound 1 and Compound 3. Hence, AFDX0250 in higher yield and higher purity can be prepared by using the purification method of the present invention.

Example 5: Purification of AFDX0250 (2)

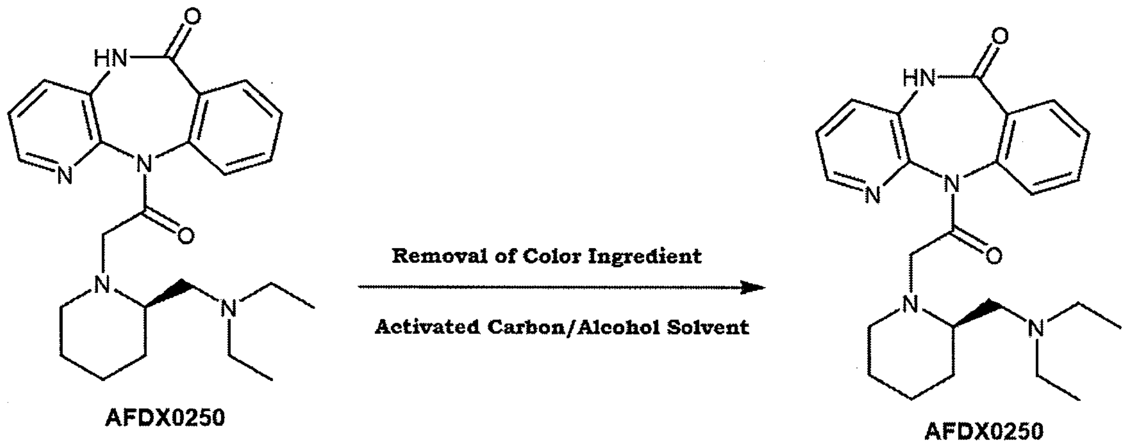


AFDX0250 AFDX0250

For the purpose of ensuring proper appearance and quality of pharmaceutical products, it is necessary to control color which is one of the items indicating the appearance of drug substances in the process of manufacturing drug substances. The crude AFDX0250 was dissolved in an organic solvent with heating, an activated carbon was added thereto and the mixture was stirred, the activated carbon was removed by filtration and the filtrate was decolorized, and colored ingredients were removed to purify AFDX0250.

In this study, each sample was prepared using Shirasagi A (0.05 w/w, 0.10 w/w and 0.15 w/w) and Shirasagi P (0.10 w/w and 0.15 w/w) as activated carbon and ethanol (EtOH), 1-propanol (1-PrOH) and N-methylpyrrolidone (NMP) as organic solvent. The samples comprising NMP as solvent treated with Shirasagi A (0.15 w/w) and Shirasagi P (0.10 w/w and 0.15 w/w) are not prepared.

The amounts of ethanol and 1-propanol added were 20 mL per g of the crude AFDX0250, respectively, and the stirring temperature was 70 °C. The amount of NMP added was 2.5 mL per g of the crude AFDX0250 and the stirring temperature was 70 °C.

In order to confirm whether color ingredients are removed, the properties of the samples comprising ethanol, 1-propanol and NMP before and after the treatment with activated carbon were confirmed. In addition, the HPLC analysis was performed for each sample at each point (before the addition of activated carbon, 1 hour after the addition of activated carbon, 3 hours after the addition of activated carbon).

The appearances of each sample (powder and DMSO solution) before and after the treatment with activated carbon are shown in Fig. 1. The appearances of each powder were confirmed by filtrating each sample comprising each organic solvent *in vacuo,* concentrating the resulting filtrate *in vacuo,* drying the resulting wet solid *in vacuo* at 50 °C, and the appearances of each DMSO solution were confirmed by dissolving the resulting solid in 10% dimethyl sulfoxide (DMSO).

In addition, the HPLC analysis results of the sample comprising ethanol (EtOH), the sample comprising 1-propanol (1-PrOH) and the sample comprising NMP are shown in Table 10 to Table 12.

[Table 10]

| Sample comprising ethanol | | | |
|---|---|---|---|
| Activated carbon | Measurement sample | HPLC (Area%) | |
| | | AFDX0250 | Compound 3 |
| Shirasagi A (0.05 w/w) | Before addition of activated carbon | 97.28% | 1.96% |
| | 1hr after addition of activated carbon | 97.75% | 1.38% |
| | 3hr after addition of activated carbon | 97.64% | 1.42% |
| Shirasagi A (0.10 w/w) | Before addition of activated carbon | 97.25% | 1.97% |
| | 1hr after addition of activated carbon | 98.20% | 0.93% |
| | 3hr after addition of activated carbon | 98.04% | 1.01% |
| Shirasagi A (0.15 w/w) | Before addition of activated carbon | 97.19% | 1.97% |
| | 1hr after addition of activated carbon | 98.60% | 0.60% |
| | 3hr after addition of activated carbon | 98.50% | 0.63% |

(continued)

| Sample comprising ethanol | | | |
|---|---|---|---|
| Activated carbon | Measurement sample | HPLC (Area%) | |
| | | AFDX0250 | Compound 3 |
| Shirasagi P (0.10 w/w) | Before addition of activated carbon | 96.93% | 1.97% |
| | 1hr after addition of activated carbon | 97.85% | 0.87% |
| | 3hr after addition of activated carbon | 97.65% | 0.92% |
| Shirasagi P (0.15 w/w) | Before addition of activated carbon | 97.22% | 1.96% |
| | 1hr after addition of activated carbon | 97.76% | 0.56% |
| | 3hr after addition of activated carbon | 97.62% | 0.61% |

[Table 11]

| Sample comprising 1-propanol | | | |
|---|---|---|---|
| Activated carbon | Measurement sample | HPLC (Area%) | |
| | | AFDX0250 | Compound 3 |
| Shirasagi A (0.05 w/w) | Before addition of activated carbon | 97.30% | 1.95% |
| | 1hr after addition of activated carbon | 97.72% | 1.43% |
| | 3hr after addition of activated carbon | 97.79% | 1.44% |
| Shirasagi A (0.10 w/w) | Before addition of activated carbon | 97.04% | 1.98% |
| | 1hr after addition of activated carbon | 98.14% | 0.96% |
| | 3hr after addition of activated carbon | 98.17% | 1.03% |
| Shirasagi A (0.15 w/w) | Before addition of activated carbon | 97.23% | 1.95% |
| | 1hr after addition of activated carbon | 98.49% | 0.68% |
| | 3hr after addition of activated carbon | 98.46% | 0.72% |
| Shirasagi P (0.10 w/w) | Before addition of activated carbon | 96.96% | 1.94% |
| | 1hr after addition of activated carbon | 97.36% | 0.91% |
| | 3hr after addition of activated carbon | 97.77% | 0.91% |
| Shirasagi P (0.15 w/w) | Before addition of activated carbon | 96.86% | 1.92% |
| | 1hr after addition of activated carbon | 97.94% | 0.58% |
| | 3hr after addition of activated carbon | 97.86% | 0.64% |

[Table 12]

| Sample comprising NMP | | | |
|---|---|---|---|
| Activated carbon | Measurement sample | HPLC (Area%) | |
| | | AFDX0250 | Compound 3 |
| Shirasagi A (0.05 w/w) | Before addition of activated carbon | 96.98% | 1.99% |
| | 1hr after addition of activated carbon | 96.83% | 2.12% |
| | 3hr after addition of activated carbon | 96.11% | 2.59% |
| Shirasagi A (0.10 w/w) | Before addition of activated carbon | 97.05% | 2.02% |
| | 1hr after addition of activated carbon | 96.81% | 2.17% |
| | 3hr after addition of activated carbon | 96.08% | 2.63% |

In this study, it was shown that color ingredients and Compound 3, which is an impurity, could be sufficiently removed by the treatment with activated carbon using an alcohol solvent.

Example 6: Purification of AFDX0250 (3)

To a solution of the crude AFDX0250 (200 g, 0.474 mol) purified in Example 4 in ethanol (3200 g) was added activated carbon, Shirasagi A (30.0 g) at room temperature under nitrogen atmosphere, the mixture was heated and stirred at an internal temperature of 69.0 to 72.4 °C for 1 hour, and then the mixture was filtrated *in vacuo* using a filtration device filled with filer aid, and the filtrate was washed with ethanol (320 g) preheated to 70 °C. The filtrate was concentrated *in vacuo* to obtain concentrated residue (989.04 g).

In this study, in order to perform the recrystallization process with the treatment process with activated carbon from the viewpoint of the work efficiency of the purification process, the recrystallization process by a combination of the alcohol used in the treatment with activated carbon and a poor solvent was performed.

Ethanol or 1-propanol was used as the alcohol solvent and acetone, methyl ethyl ketone (MEK), methyl isobutyl ketone (MIBK) which are ketone solvents or n-heptane was used as the poor solvent. The recrystallization with each combination of the alcohol solvent and the poor solvent at the total solvent amounts of 10 v/w (Ratio 1:3) and 20 v/w (Ratio 1:1) was performed as follows.

A solution of AFDX0250 in an alcohol solvent was heated and stirred at 50 °C for 0.5 hour to 1 hour, each poor solvent was added to the solution, and then the mixture was heated and stirred for additional 1 hour. The mixture was then cooled to 5 °C and aged for 24 hours. The suspension was filtrated and the filtrated product was dried *in vacuo* at 90 °C. The appearance of the resulting dried product was confirmed and the HPLC analysis was performed for the dried product. Also, the dried product was prepared in 10% dimethyl sulfoxide (DMSO) solution and the appearance of the solution was confirmed.

The properties of each dried product obtained in the recrystallization process using ethanol and 1-propanol as alcohol solvent and DMSO solution prepared therefrom as well as the appearances of dried product before the purification and DMSO solution prepared therefrom are shown in Fig. 2 and Fig. 3, respectively. In addition, the HPLC analysis results of each dried product obtained in the recrystallization process using ethanol and 1-propanol as alcohol solvent as well as dried product before the purification and the yield thereof are shown in Table 13 and Table 14, respectively.

[Table 13]

| Alcohol solvent: Ethanol | | | | | |
|---|---|---|---|---|---|
| Poor solvent | Poor solvent/Alcohol solvent | Total solvent amount | HPLC (Area%) | | Yield |
| | | | AFDX0250 | Compound 1 | |
| Acetone | 3 | 10 v/w | 98.98% | 0.77% | 70.8% |
| | 1 | 20 v/w | 98.59% | 1.07% | 54.6% |
| MEK | 3 | 10 v/w | 99.17% | 0.68% | 72.9% |
| | 1 | 20 v/w | 98.51% | 1.23% | 57.6% |
| MIBK | 3 | 10 v/w | 99.46% | 0.49% | 77.0% |
| | 1 | 20 v/w | 98.49% | 1.24% | 67.8% |
| n-Heptane | 3 | 10 v/w | 97.62% | 1.92% | 88.3% |
| | 1 | 20 v/w | 98.40% | 1.47% | 78.1% |
| Before purification | | | 94.80% | 3.71% | |

[Table 14]

| Alcohol solvent: 1-Propanol | | | | | |
|---|---|---|---|---|---|
| Poor solvent | Poor solvent/Alcohol solvent | Total solvent amount | HPLC (Area%) | | Yield |
| | | | AFDX0250 | Compound 1 | |
| Acetone | 3 | 10 v/w | 99.12% | 0.67% | 74.1% |
| | 1 | 20 v/w | 98.78% | 0.95% | 59.0% |

(continued)

| Alcohol solvent: 1-Propanol | | | | | |
|---|---|---|---|---|---|
| Poor solvent | Poor solvent/Alcohol solvent | Total solvent amount | HPLC (Area%) | | Yield |
| | | | AFDX0250 | Compound 1 | |
| MEK | 3 | 10 v/w | 99.50% | 0.42% | 75.7% |
| | 1 | 20 v/w | 98.99% | 0.88% | 54.3% |
| MIBK | 3 | 10 v/w | 99.00% | 0.78% | 84.3% |
| | 1 | 20 v/w | 98.82% | 0.96% | 66.9% |
| n-Heptane | 3 | 10 v/w | 96.93% | 2.55% | 93.9% |
| | 1 | 20 v/w | 98.09% | 1.57% | 80.9% |
| Before purification | | | 95.27% | 3.33% | |

In this study, it was shown that color ingredients and Compound 1, which is an impurity, could be sufficiently removed and the purity of AFDX0250 was enhanced by performing the recrystallization process using a mixed solvent of an alcohol solvent and a ketone solvent.

<u>Example 7: Purification of AFDX0250 (4)</u>

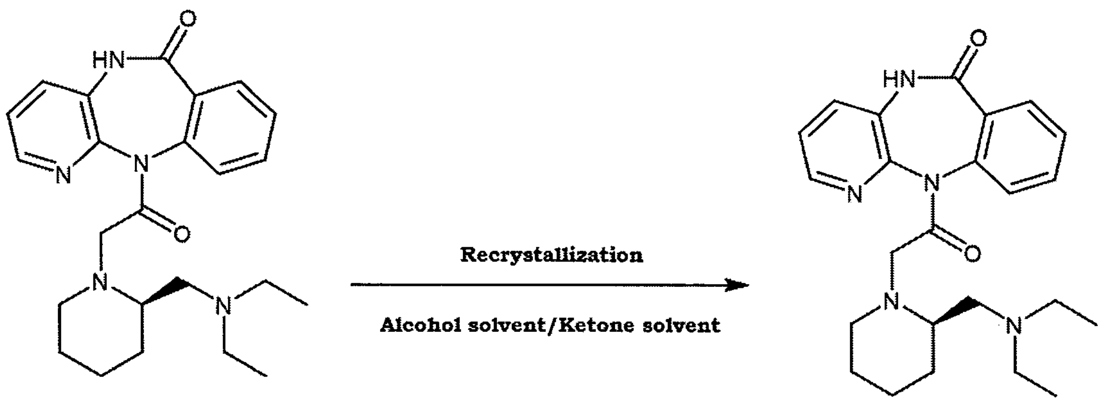


AFDX0250 AFDX0250

To a solution of AFDX0250 treated with activated carbon in Example 6 in ethanol was added methyl isobutyl ketone (MIBK) (640 g) and the mixture was concentrated. The mass volume percentages of ethanol and MIBK to AFDX0250 was calculated by the $^{1}$H-NMR measurement and the amount of ethanol was adjusted so that the total amount of ethanol is 2.5 v/w. The reaction mixture was then heated to 63 to 67 °C and stirred for 30 minutes. MIBK was added dropwise over 48 minutes so that the total amount of MIBK is 7.5 v/w, and the mixture was stirred at 63 to 67 °C for 1 hour. The mixture was then cooled to 0 to 5 °C, aged for 24 hours and filtrated to obtain a crystal (1st recrystallization).

According to (1) Measurement method of amount of AFDX0250 or a salt thereof as described herein, sample solution was prepared using the resulting AFDX0250, and the HPLC analysis was performed for the sample solution.

In addition, the resulting AFDX0250 was dissolved in ethanol and the solution was subjected to 2nd recrystallization in a similar manner to the 1st recrystallization. The resulting wet solid AFDX0250 was dried at 90 °C for 14 hours and 35 minutes to obtainAFDX0250 (152.27 g, Yield 76.1%).

According to (1) Measurement method of amount of AFDX0250 or a salt thereof as described herein, sample solution was prepared using the resulting AFDX0250 as with AFDX0250 obtained by the 1st recrystallization, and the HPLC analysis was performed for the sample solution.

The $^{1}$ H-NMR data of AFDX0250 is as follows.

$^{1}$ H-NMR (400 MHz, $CDCl_3$) δ 0.85-0.97 (m, 6H), 1.08-1.75 (m, 6H), 1.96-2.60 (m, 7.5H), 2.72-2.82 (m, 0.5H), 3.10-3.24 (m, 0.5H), 3.68 (s, 1H), 4.12-4.26 (m, 0.5H), 7.27-7.33 (m, 1H), 7.36-7.43 (m, 1H), 7.59-7.67 (m, 3H), 7.93 (d,J=7.8Hz, 1H), 8.30 (d,J=3.2Hz, 1H), 10.00-11.00 (br,1H)

The HPLC analysis results of AFDX0250 obtained by the 1st and 2nd recrystallization are shown in Table 15. The term "N.D." in the table means "Not Detected" and the "a)" shows the amount of Compound 1 measured by liquid chromatography mass spectrometry.

[Table 15]

| Measurement object | AFDX0250 | Compound 1 | Compound 3 | Compound 5 |
|---|---|---|---|---|
| Crude AFDX0250 purified | 99.47% | 1.1 ppm | 0.03% | 0.22% |
| AFDX0250 after 1st recrystallization | 99.67% | N.D. | 0.01% | 0.08% |
| AFDX0250 after 2nd recrystallization | 99.80% | N.D. (1.19 ppm)[a)] | 0.01% | 0.03% |

In this study, it was shown that Compound 5 could be sufficiently removed by the recrystallization after the treatment with activated carbon as with Compound 1. Hence, it was shown that the purity of AFDX0250 was further enhanced by the combination of the treatment with activated carbon and the recrystallization.

INDUSTRIAL APPLICABILITY

According to the present invention, (R)-11-[2-[2-[(diethyl)methyl]-1-piperidinyl]acetyl]-5,11-dihydro-6H-pyrido[2,3-b][1,4]benzodiazepin-6-one or a salt thereof in higher yield and higher purity can be prepared as compared to the known methods. Also, the present invention can reduce the amounts of impurities such as 11-(2-chloroacetyl)-5,11-dihydro-6H-pyrido[2,3-b][1,4]benzodiazepin-6-one.

In addition, the present invention can provide (R)-11-[2-[2-[(diethyl)methyl]-1-piperidinyl]acetyl]-5,11-dihydro-6H-pyrido[2,3-b][1,4]benzodiazepin-6-one or a salt thereof in high purity and a pharmaceutical composition comprising (R)-11-[2-[2-[(diethyl)methyl]-1-piperidinyl]acetyl]-5,11-dihydro-6H-pyrido[2,3-b][1,4]benzodiazepin-6-one or a salt thereof in high purity.

## Claims

1. A method of preparing (R)-11-[2-[2-[(diethylamino)methyl]-1-piperidinyl]acetyl]-5,11-dihydro-6H-pyrido[2,3-b][1,4]benzodiazepin-6-one or a salt thereof, which comprises reacting 11-(2-chloroacetyl)-5,11-dihydro-6H-pyrido[2,3-b][1,4]benzodiazepin-6-one or a salt thereof and (R)-2-(diethylamino)methylpiperidine or a salt thereof in an organic solvent under a basic condition in the presence of an alkali metal iodide, wherein the organic solvent comprises acetonitrile in an amount of 15 to 50 mL per g of 11-(2-chloroacetyl)-5,11-dihydro-6H-pyrido[2,3-b][1,4]benzodiazepin-6-one or a salt thereof.

2. The method according to claim 1, wherein the organic solvent is acetonitrile in an amount of 15 to 50 mL per g of 11-(2-chloroacetyl)-5,11-dihydro-6H-pyrido[2,3-b][1,4]benzodiazepin-6-one or a salt thereof.

3. The method according to claim 1 or 2, which comprises adding water and an acid to the resulting reaction mixture to adjust the pH thereof and then extracting the reaction mixture with an organic solvent.

4. The method according to claim 3, wherein the extraction with an organic solvent is performed twice or more.

5. The method according to claim 3 or 4, wherein the organic solvent is ethyl acetate.

6. A method of preparing (R)-11-[2-[2-[(diethylamino)methyl]-1-piperidinyl]acetyl]-5,11-dihydro-6H-pyrido[2,3-b][1,4]benzodiazepin-6-one or a salt thereof, which comprises reacting 11-(2-iodoacetyl)-5,11-dihydro-6H-pyrido[2,3-b][1,4]benzodiazepin-6-one or a salt thereof and (R)-2-(diethylamino)methylpiperidine or a salt thereof in an organic solvent under a basic condition.

7. A method of purifying (R)-11-[2-[2-[(diethylamino)methyl]-1-piperidinyl]acetyl]-5,11-dihydro-6H-pyrido[2,3-b][1,4]benzodiazepin-6-one or a salt thereof, which comprises crystallizing (R)-11-[2-[2-[(diethylamino)methyl]-1-piperidinyl]acetyl]-5,11-dihydro-6H-pyrido[2,3-b][1,4]benzodiazepin-6-one or a salt thereof in a mixture of 1-propanol and water.

8. A method of purifying (R)-11-[2-[2-[(diethylamino)methyl]-1-piperidinyl]acetyl]-5,11-dihydro-6H-pyrido[2,3-b][1,4]benzodiazepin-6-one or a salt thereof, which comprises treating (R)-11-[2-[2-[(diethylamino)methyl]-1-piperidinyl]acetyl]-5,11-dihydro-6H-pyrido[2,3-b][1,4]benzodiazepin-6-one or a salt thereof with an activated carbon in an alcohol solvent.

**9.** A method of purifying (R)-11-[2-[2-[(diethylamino)methyl]-1-piperidinyl]acetyl]-5,11-dihydro-6H-pyrido[2,3-b][1,4]benzodiazepin-6-one or a salt thereof, which comprises recrystallizing (R)-11-[2-[2-[(diethylamino)methyl]-1-piperidinyl]acetyl]-5,11-dihydro-6H-pyrido[2,3-b][1,4]benzodiazepin-6-one or a salt thereof in a mixture of an alcohol solvent and a ketone solvent.

**10.** (R)-11-[2-[2-[(diethylamino)methyl]-1-piperidinyl]acetyl]-5,11-dihydro-6H-pyrido[2,3-b][1,4]benzodiazepin-6-one or a salt thereof with a purity of 95% or more.

**11.** (R)-11-[2-[2-[(diethylamino)methyl]-1-piperidinyl]acetyl]-5,11-dihydro-6H-pyrido[2,3-b][1,4]benzodiazepin-6-one or a salt thereof, wherein an amount of impurities therein is less than 5%.

**12.** A pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt, wherein the pharmaceutical composition comprises impurities derived from (R)-11-[2-[2-[(diethylamino)methyl]-1-piperidinyl]acetyl]-5,11-dihydro-6H-pyrido[2,3-b][1,4]benzodiazepin-6-one or a salt thereof in an amount of less than 5% relative to (R)-11-[2-[2-[(diethylamino)methyl]-1-piperidinyl]acetyl]-5,11-dihydro-6H-pyrido[2,3-b][1,4]benzodiazepin-6-one or a salt thereof.

**13.** The pharmaceutical composition according to claim 12, wherein the amount of the impurities is less than 1%.

**14.** A pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the pharmaceutical composition is substantially free of 11-(2-chloroacetyl)-5,11-dihydro-6H-pyrido[2,3-b][1,4]benzodiazepin-6-one or a salt thereof.

**15.** The pharmaceutical composition according to claim 14, wherein the amount of 11-(2-chloroacetyl)-5,11-dihydro-6H-pyrido[2,3-b][1,4]benzodiazepin-6-one is less than 1000 ppm relative to (R)-11-[2-[2-[(diethylamino)methyl]-1-piperidinyl]acetyl]-5,11-dihydro-6H-pyrido[2,3-b][1,4]benzodiazepin-6-one or a salt thereof.

**16.** The pharmaceutical composition according to claim 14 or 15, wherein the pharmaceutical composition is substantially free of 5,11-dihydro-6H-pyrido[2,3-b][1,4]benzodiazepin-6-one or a salt thereof.

**17.** The pharmaceutical composition according to claim 16, wherein the amount of 5,11-dihydro-6H-pyrido[2,3-b][1,4]benzodiazepin-6-one or a salt thereof is less than 1000 ppm.

[Fig. 1]

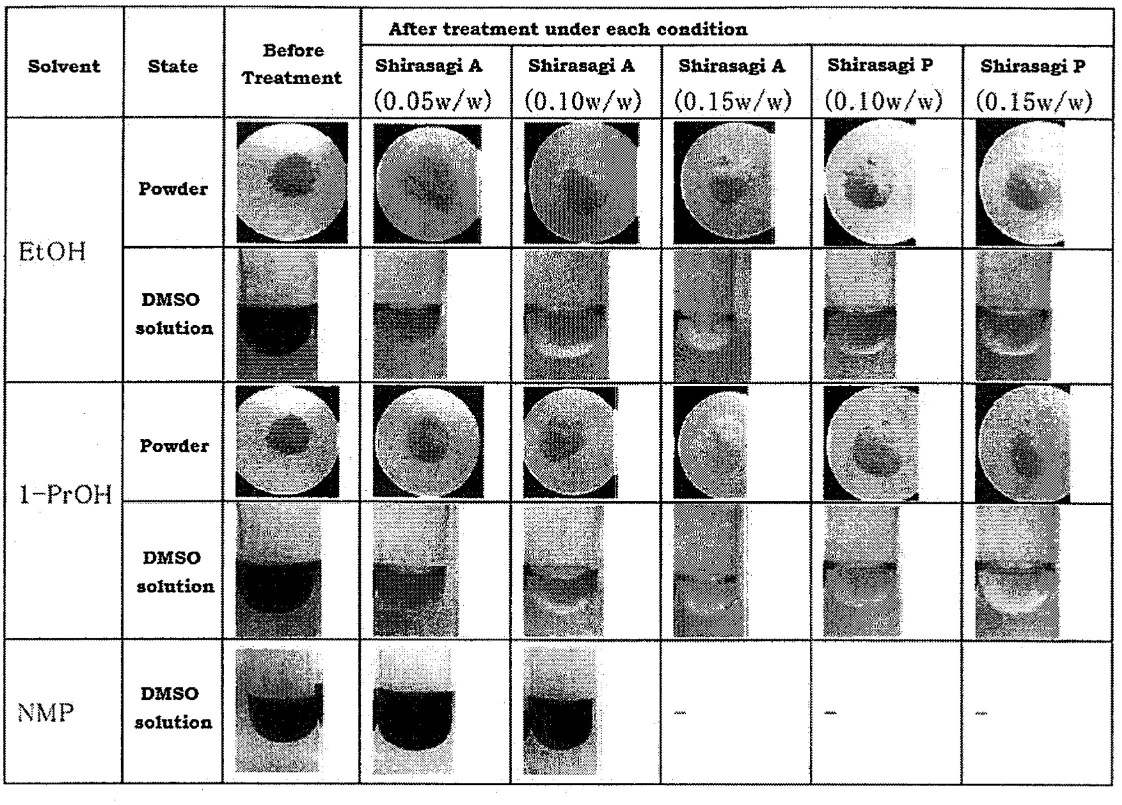
Solvent
State
Before Treatment
After treatment under each condition
Shirasagi A (0.05w/w)
Shirasagi A (0.10w/w)
Shirasagi A (0.15w/w)
Shirasagi P (0.10w/w)
Shirasagi P (0.15w/w)
EtOH
Powder
DMSO solution
1-PrOH
Powder
DMSO solution
NMP
DMSO solution
-
-
-

[Fig. 2]

| Poor solvent | Poor solvent/Alcohol solvent (Ethanol) | Total solvent amount | Powder | DMSO solution |
|---|---|---|---|---|
| Acetone | 3 | 10v/w | 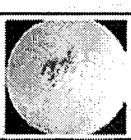 | 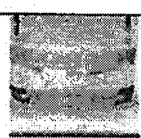 |
| | 1 | 20v/w | 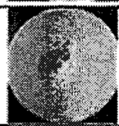 | 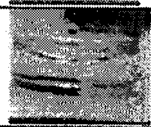 |
| MEK | 3 | 10v/w | 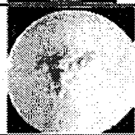 | 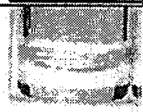 |
| | 1 | 20v/w | 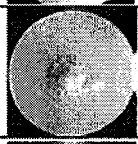 | 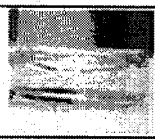 |
| MIBK | 3 | 10v/w | 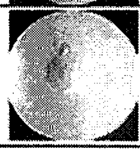 | 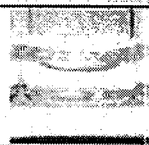 |
| | 1 | 20v/w | 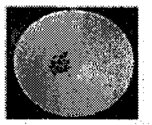 | 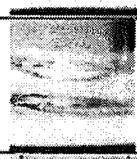 |
| n-Heptane | 3 | 10v/w | 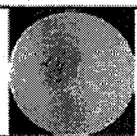 | 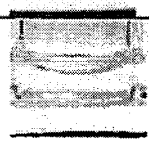 |
| | 1 | 20v/w | 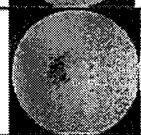 | 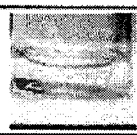 |
| Before purification | | | 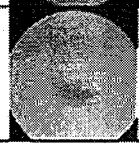 | 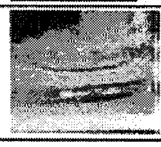 |

[Fig. 3]

| Poor solvent | Poor solvent/Alcohol solvent (1-Propanol) | Total solvent amount | Powder | DMSO solution |
|---|---|---|---|---|
| Acetone | 3 | 10v/w | 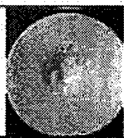 | 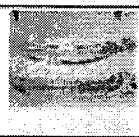 |
| | 1 | 20v/w | 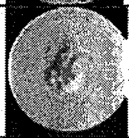 | 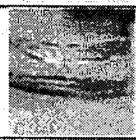 |
| MEK | 3 | 10v/w | 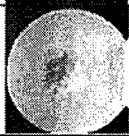 | 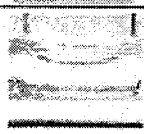 |
| | 1 | 20v/w | 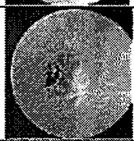 | 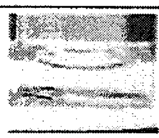 |
| MIBK | 3 | 10v/w | 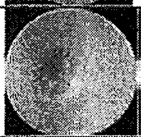 | 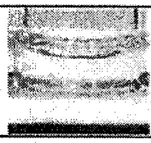 |
| | 1 | 20v/w | 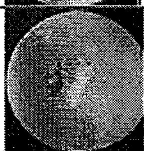 | 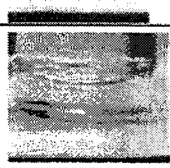 |
| n-Heptane | 3 | 10v/w | 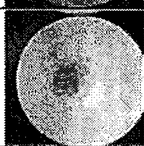 | 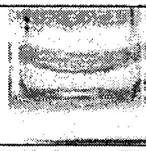 |
| | 1 | 20v/w | 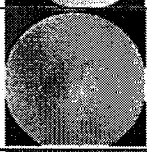 | 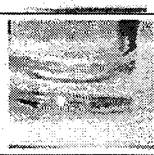 |
| Before purification | | | 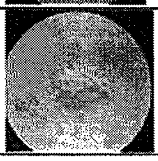 | 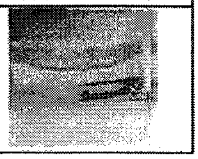 |

**INTERNATIONAL SEARCH REPORT**

International application No.
**PCT/JP2023/012761**

**A. CLASSIFICATION OF SUBJECT MATTER**

***C07D 471/04***(2006.01)i; ***A61K 31/5513***(2006.01)i; ***A61P 27/02***(2006.01)i; ***A61P 27/06***(2006.01)i
FI: C07D471/04 121; A61K31/5513; A61P27/02; A61P27/06; C07D471/04 CSP

According to International Patent Classification (IPC) or to both national classification and IPC

**B. FIELDS SEARCHED**

Minimum documentation searched (classification system followed by classification symbols)

C07D471/04; A61K31/5513; A61P27/02; A61P27/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | J. Med. Chem. 1989, v. 32, pp. 1718-1724<br>p. 1721, right column, etc. | 1-5 |
| Y | JP 60-215683 A (DR KARL THOMAE GMBH) 29 October 1985 (1985-10-29)<br>example 52, etc. | 1–5 |
| Y | Chem. Pharm. Bull. 2000, v. 48, pp. 1611-1622<br>chart 1 and p. 1618, lower right, etc. | 1-5 |
| Y | WO 2016/078770 A1 (LABORATORIOS DEL DR. ESTEVE, S. A.) 26 May 2016 (2016-05-26)<br>example 126, etc. | 1-5 |
| A | JP 7-504915 A (ALLERGAN, INCORPORATED) 01 June 1995 (1995-06-01)<br>p. 5, lower right column, etc. | 1-5 |
| A | WO 2022/030489 A1 (SANTEN PHARMACEUTICAL CO., LTD.) 10 February 2022 (2022-02-10)<br>paragraph [0001], etc. | 1-5 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 June 2023** | **04 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/012761**

**C. DOCUMENTS CONSIDERED TO BE RELEVANT**

| Category* | Citation of document, with indication, where appropriate, of the relevant passages | Relevant to claim No. |
|---|---|---|
| A | JP 63-35573 A (DR KARL THOMAE GMBH) 16 February 1988 (1988-02-16)<br>entire text | 1–5 |
| A | Chem. Pharm. Bull. 1999, v. 47, pp. 672-677<br>entire text | 1–5 |
| A | Chem. Pharm. Bull. 1997, v. 45, pp. 1458-1469<br>entire text | 1–5 |

Form PCT/ISA/210 (second sheet) (January 2015)

INTERNATIONAL SEARCH REPORT

International application No.
PCT/JP2023/012761

**Box No. III Observations where unity of invention is lacking (Continuation of item 3 of first sheet)**

This International Searching Authority found multiple inventions in this international application, as follows:

Claim 1, which is an independent claim, and claims 2-5 citing claim 1 set forth a method for manufacturing (R)-11-[2-[2-[(diethylamino)methyl]-1-piperidinyl]acetyl-5,11-dihydro-6H-pyrido[2,3-b][1,4]benzodiazepine-6-on (citing paragraph [0001] of the present specification, hereinafter referred to as "AFDX0250") or a salt thereof, the method being characterized in that, in particular, production is carried out in the presence of an alkali metal iodide and a specific amount of acetonitrile.

Claim 6, which is an independent claim, sets forth a method for manufacturing AFDX0250 or a salt thereof, the method being characterized in that, in particular, manufacturing is carried out using 11-(2-iodoacetyl)-5,11-dihydro-6H-pyrido[2,3-b][1,4]benzodiazepine-6-on.

Claim 7, which is an independent claim, sets forth a method for refining AFDX0250 or a salt thereof, the method being characterized in that, in particular, crystallization is carried out in a specific mixed solution.

Claim 8, which is an independent claim, sets forth a method for refining AFDX0250 or a salt thereof, the method being characterized in that, in particular, an activated carbon treatment is carried out.

Claim 9, which is an independent claim, sets forth a method for refining AFDX0250 or a salt thereof, the method being characterized in that, in particular, re-crystallizing is performed in a specific mixed solution (note that this mixed solution is different from the mixed solution of the invention in claim 7).

Claim 10, which is an independent claim, sets forth a compound which is AFDX0250 or a salt thereof, the compound being characterized in that, in particular, the degree of purity of AFDX0250 is specified.

Claim 11, which is an independent claim, sets forth a compound which is AFDX0250 or a salt thereof, characterized by, in particular, specifying the amount of impurities.

Claim 12, which is an independent claim, and claim 13 citing claim 12 set forth a pharmaceutical composition containing AFDX0250 or a salt thereof, characterized by, in particular, specifying the amount of impurities.

Claim 14, which is an independent claim, and claims 15-17 citing claim 14 set forth a pharmaceutical composition containing AFDX0250 or a salt thereof, the composition being characterized by, in particular,being specified by the amount of 11-(2-chloroacetyl)-5,11-dihydro-6H-pyrido[2,3-b][1,4]benzodiazepine-6-on or a salt thereof, or 5,11-dihydro-6H-pyrido[2,3-b][1,4]benzodiazepine-6-on or a salt thereof.

Therefore, the inventions in claims 1-17 of the present application are considered to share only the feature of being an invention relating to "AFDX0250 or a salt thereof". However, in the light of the fact that document 1 (compound 29 on page 1721), document 2 (example 52), document 5 (page 5, lower right column), document 6 (paragraph [0001]), etc., specifically disclose AFDX0250 or a salt thereof, the aforementioned feature does not make a contribution over the prior art and thus cannot be said to be a special technical feature. Furthermore, there are no other identical or corresponding special technical features between claims 1-17.

Accordingly, the invention as in claims 1-17 of the present application is classified into the following five inventions.

(Invention 1) The inventions in claim 1 and in claims 2-5 citing claim 1
(Invention 2) The invention in claim 6
(Invention 3) The inventions in claims 7-9
(Invention 4) The inventions in claims 10-11
(Invention 5) The inventions in claims 12-17

Document 1: J. Med. Chem. 1989, v. 32, pp. 1718-1724
Document 2: JP 60-215683 A
Document 5: JP 7-504915 A
Document 6: WO 2022/030489 A1

INTERNATIONAL SEARCH REPORT

International application No.
**PCT/JP2023/012761**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-5**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2023/012761**

| Patent document cited in search report | Publication date (day/month/year) | Patent family member(s) | Publication date (day/month/year) |
|---|---|---|---|
| JP 60-215683 A | 29 October 1985 | US 4550107 A<br>example 52, etc.<br>EP 156191 A2<br>KR 10-1985-0006403 A | |
| WO 2016/078770 A1 | 26 May 2016 | JP 2017-535545 A<br>US 2018/0111935 A1<br>EP 3221314 A1<br>CN 107001369 A | |
| JP 7-504915 A | 01 June 1995 | US 5716952 A<br>WO 1993/018772 A1<br>EP 634932 A1 | |
| WO 2022/030489 A1 | 10 February 2022 | JP 2022-539639 A<br>CN 114302726 A | |
| JP 63-35573 A | 16 February 1988 | US 4971966 A<br>EP 254955 A2<br>KR 10-1988-0001653 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9318772 A
- WO 2022030489 A
- JP S60215683 A


### Non-patent literature cited in the description


- **WOLFNARD W. ENGEL et al.** *J. Med. Chem.*, 1989, vol. 32 (8), 1718-1724